(19) Europäisches Patentamt
European Patent Office
Office européen des brevets

(11) EP 2 261 206 A1

(12) **EUROPEAN PATENT APPLICATION**
published in accordance with Art. 153(4) EPC

(43) Date of publication:
**15.12.2010 Bulletin 2010/50**

(21) Application number: **09729006.8**

(22) Date of filing: **30.03.2009**

(51) Int Cl.:
***C07D 209/34*** (2006.01) ***A61K 31/4045*** (2006.01)
***A61K 31/405*** (2006.01) ***A61K 31/4178*** (2006.01)
***A61K 31/427*** (2006.01) ***A61K 31/4439*** (2006.01)
***A61K 31/5377*** (2006.01) ***A61P 1/04*** (2006.01)
***A61P 1/14*** (2006.01) ***C07D 401/06*** (2006.01)
***C07D 403/06*** (2006.01) ***C07D 405/06*** (2006.01)
***C07D 417/06*** (2006.01)

(86) International application number:
**PCT/JP2009/056431**

(87) International publication number:
**WO 2009/123080 (08.10.2009 Gazette 2009/41)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **01.04.2008 JP 2008095040**

(71) Applicant: **Astellas Pharma Inc.**
**Tokyo 103-8411 (JP)**

(72) Inventors:
- **KAKU, Hidetaka**
**Tokyo 103-8411 (JP)**
- **TAKUWA, Tomofumi**
**Tokyo 103-8411 (JP)**
- **KAGEYAMA, Michihito**
**Tokyo 103-8411 (JP)**
- **NOZAWA, Katsura**
**Tokyo 103-8411 (JP)**
- **DOIHARA, Hitoshi**
**Tokyo 103-8411 (JP)**

(74) Representative: **Hiebl, Inge Elisabeth**
**Kraus & Weisert**
**Patent- und Rechtsanwälte**
**Thomas-Wimmer-Ring 15**
**80539 München (DE)**

(54) **INDOLINONE COMPOUND**

(57) [Problems] A compound, which is useful as an active ingredient for a pharmaceutical composition, for example a pharmaceutical composition for treating constipation-type irritable bowel syndrome, atonic constipation and/or functional gastrointestinal disorder, is provided.

[Means for Solution] The present inventors have extensively studied compounds having TRPA1 channel activation activity, and confirmed that an indolinone compound has a TRPA1 channel activation activity, and thus completed the present invention. The indolinone compound of the present invention has a TRPA1 channel activation activity and can be used as an active ingredient of a pharmaceutical composition for preventing and/or treating constipation-type irritable bowel syndrome, atonic constipation and/or functional gastrointestinal disorder or the like.



Printed by Jouve, 75001 PARIS (FR)

## Description

### Technical Field

The present invention relates to an indolinone compound which is useful as an active ingredient for a pharmaceutical composition, for example a pharmaceutical composition for treating constipation-type irritable bowel syndrome (constipation-type IBS), atonic constipation and/or functional gastrointestinal disorder.

### Background Art

90% of serotonin (hereinafter, referred to "5-HT") in the living body is present in the gastrointestinal tract. 5-HT in the gastrointestinal tract is biosynthesized in enterochromaffm cells (hereinafter, referred to "EC cell") of the gastrointestinal tract mucosa, then enters into the blood, and is transferred to the whole body. The 5-HT released by a chemical stimulation or mechanical stimulation to the intestinal tract bonds to 5-HT receptors of target cells and causes a physiological response. As 5-HT receptors involved in gastrointestinal tract motility function, 5-HT receptor 1, 5-HT receptor 2, 5-HT receptor 3, 5-HT receptor 4, 5-HT receptor 7, and the like have been recognized. It has been found that these receptors are expressed in nerve cells or the smooth muscle of the gastrointestinal tract. The 5-HT released from EC cells controls gastrointestinal tract motility function through the nerve cells or smooth muscle which expresses these 5-HT receptors. In other words, 5-HT is considered to be a kind of hormone which controls gastrointestinal tract function (Non Patent Document 1).

It has been known for some time that, when a chemical stimulation or mechanical stimulation is given to EC cells, release of 5-HT is promoted and intestinal motility is increased. However, it has not been identified which molecular mechanism causes on the promotion of the release of 5-HT from the EC cells as mentioned above.

At present, a medicine for controlling 5-HT receptor activity is used in clinical practice in the gastrointestinal tract disorder area. For example, a 5-HT receptor 3 inhibitor is used in treatment of diarrhea type IBS or as an antiemetic agent, or the like, a 5-HT receptor 4 activating agent is used in treatment of constipation type IBS or gastrointestinal incompetence, or the like. Furthermore, it has been known that, since most of the constipation type IBS patients have a reduced amount of 5-HT in the blood, 5-HT is involved in the clinical condition (Non Patent Document 2 and Non Patent Document 3). At present, there is demand for a therapeutic medicine which gives patients high satisfaction in terms of gastrointestinal tract motility disorders such as constipation type IBS.

From the background as described above, the present inventors studied a gastrointestinal motility improving agent which uses a mechanism of promoting release of 5-HT from EC cells.

The compounds represented by the following formulae are known as compounds having TRPA1 channel activation activity (Non Patent Document 4 and Non Patent Document 5).

[chem. 1]

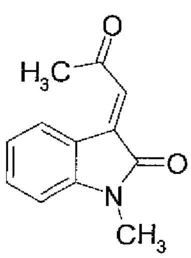

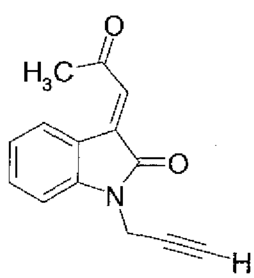

However, gastrointestinal function improving activity with respect to these compounds has not been reported. Furthermore, there is no disclosure or suggestion with respect to the compound of the formula (I) according to the present invention or a salt thereof.

Furthermore, the following compounds have been known as indolinone compounds (Non Patent Document 6).

[chem. 2]

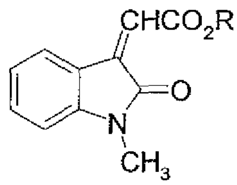
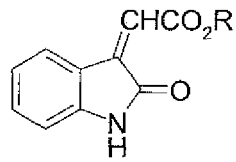

(In the formula, R represents ethyl or methyl.)
However, there is no disclosure or suggestion with respect to the compound of the formula (I) according to the present invention or a salt thereof, and furthermore there is no disclosure or suggestion with respect to TRPA1 channel activation activity or gastrointestinal function improving activity.

Furthermore, the following compound has been known as an indolinone compound (Non Patent Document 7).

[chem. 3]

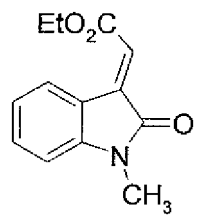

(In the formula, Et represents ethyl.)
However, there is no disclosure or suggestion with respect to the compound of the formula (I) according to the present invention or a salt thereof, and furthermore there is no disclosure or suggestion with respect to TRPA1 channel activation activity or gastrointestinal function improving activity.

Furthermore, the following compounds have been known as indolinone compounds (Non Patent Document 8).

[chem. 4]

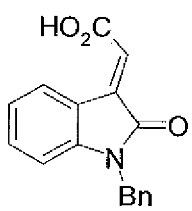
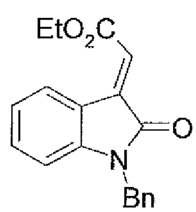
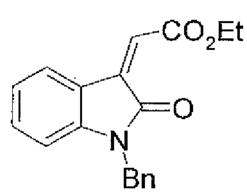

(In the formula, Bn represents benzyl and Et represents ethyl.)
However, there is no disclosure or suggestion with respect to the compound of the formula (I) according to the present invention or a salt thereof and furthermore there is no disclosure or suggestion with respect to TRPA1 channel activation activity or gastrointestinal function improving activity.

Furthermore, the following compounds have been known as indolinone compounds (Non Patent Document 9).

[chem. 5]

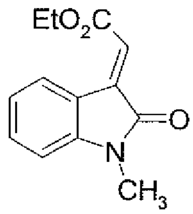

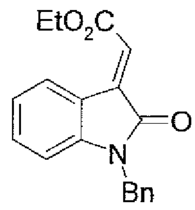

(In the formula, Et represents ethyl and Bn represents benzyl.)
However, there is no disclosure or suggestion with respect to the compound of the formula (I) according to the present invention or a salt thereof and furthermore there is no disclosure or suggestion with respect to TRPA1 channel activation activity or gastrointestinal function improving activity.

Furthermore, the following compound has been known as an indolinone compound (Patent Document 1).

[chem. 6]

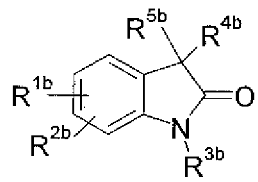

(For the symbols in the formula, refer to the document.)
However, there is no disclosure or suggestion with respect to the compound of the formula (1) according to the present invention or a salt thereof and furthermore there is no disclosure or suggestion with respect to TRPA1 channel activation activity or gastrointestinal function improving activity.

Furthermore, the following compound has been known as an indolinone compound (Patent Document 2).

[chem. 7]

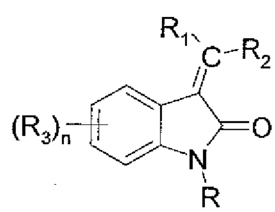

(For the symbols in the formula, refer to the document.)
However, there is no disclosure or suggestion with respect to the compound of the formula (I) according to the present invention or a salt thereof and furthermore there is no disclosure or suggestion with respect to TRPA1 channel activation activity or gastrointestinal function improving activity.

Furthermore, the following compound has been known as an indolinone compound (Patent Document 3).

[chem. 8]

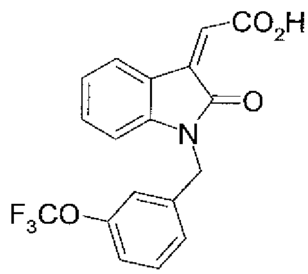

However, there is no disclosure or suggestion with respect to the compound of the formula (I) according to the present invention or a salt thereof and furthermore there is no disclosure or suggestion with respect to TRPA1 channel activation activity or gastrointestinal function improving activity.

Furthermore, the following compound has been known as an indolinone compound (Patent Document 4).

[chem. 9]

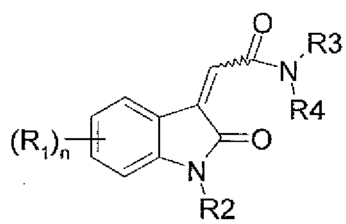

(For the symbols in the formula, refer to the document.)

However, there is no disclosure or suggestion with respect to the compound of the formula (I) according to the present invention or a salt thereof and furthermore there is no disclosure or suggestion with respect to TRPA1 channel activation activity and gastrointestinal function improving activity.

Furthermore, the following compound has been known as an indolinone compound (Non Patent Document 10).

[chem. 10]

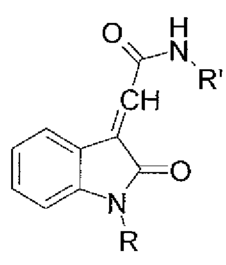

(For the symbols in the formula, refer to the document.)

However, there is no disclosure or suggestion with respect to the compound of the formula (I) according to the present invention or a salt thereof and furthermore there is no disclosure or suggestion with respect to TRPA1 channel activation activity and gastrointestinal function improving activity.

Furthermore, the following compounds have been known as indolinone compounds published after the earliest priority date of the present application (Non Patent Document 11).

[chem. 11]

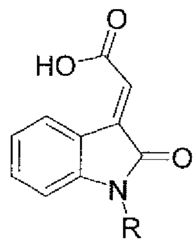 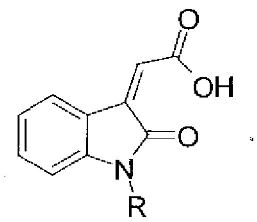

(For the symbols in the formula, refer to the document.)
However, there is no disclosure or suggestion with respect to TRPA1 channel activation activity or gastrointestinal function improving activity according to the present invention.

Related Art Documents

Patent Document 1: Pamphlet of International Publication WO2003/82265
Patent Document 2: US Patent No. 3428649
Patent Document 3: Pamphlet of International Publication WO2008/19357
Patent Document 4: Pamphlet of International Publication WO2004/56769

Non Patent Document 1: Textbook of Gastroenterolorogy, Fourth Edition, ISBN 0-7817-2861-4
Non Patent Document 2: Gastroenterology, 2006, vol. 130, p. 34-43
Non Patent Document 3: Clinical Gastroenterology and Hepatology, 2005, vol. 3, p. 349-357
Non Patent Document 4: Nature, 2007, vol. 445, p. 541-545
Non Patent Document 5: Nature, 2007, vol. 445, p. 491-492
Non Patent Document 6: Journal of Chemical Research, 2005, vol. 1, p. 62-66
Non Patent Document 7: Journal of the American Chemical Society, 1994, vol. 116, p. 9480-9486
Non Patent Document 8: Tetrahedron, 1967, vol. 23, p. 901-917
Non Patent Document 9: Tetrahedron, 2002, vol. 58, p. 7221-7231
Non Patent Document 10: Gazzetta Chimica Italiana, 1968, vol. 98, p. 344-357
Non Patent Document 11: Bioorganic and Medicinal Chemistry Letters, 2008, vol. 18, p. 3350-3353

Disclosure of the Invention

Problem that the Invention is to Solve

There is provided a compound which is useful as an active ingredient for a pharmaceutical composition, for example a pharmaceutical composition for treating constipation-type irritable bowel syndrome (constipation-type IBS), atonic constipation and/or functional gastrointestinal disorder.

Means for Solving the Problem

The present inventors studied the elucidation of a mechanism of promoting release of 5-HT from EC cells, and as a result found that TRPA1 ion channel gene is involved in release of 5-HT. The present inventors have made an intensive study on the compounds having a TRPA1 channel activation activity, have found that the compound of the formula (I) shows excellent effectiveness, and thus completed the present invention.
In other words, the present invention relates to the compound of the formula (I) or a salt thereof, and a pharmaceutical composition containing the compound of the formula (I) or a salt thereof and a pharmaceutically acceptable excipient.
[chem. 12]

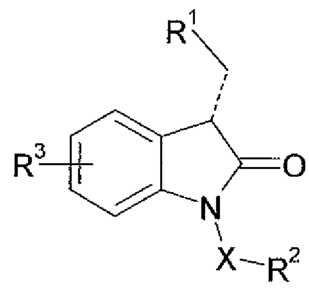

(I)

(wherein,

$R^1$ is $-CO_2H$ or a biological equivalent thereof, $-CO_2-R^0$, $-CON(-R^4)(-R^5)$, -CN, -CO-(nitrogen-containing hetero ring which may be substituted with $-R^0$), or nitrogen-containing hetero ring which may be substituted with $-R^0$,
$R^0$ is $C_{1-6}$ alkyl,
$R^4$ and $R^5$ are the same or different, representing -H, $C_{1-6}$alkyl, $C_{3-8}$ cycloalkyl, -OH, or $-SO_2-C_{1-6}$ alkyl,
X is $C_{1-10}$ alkylene, or $-(C_{1-10}$ alkylene)-O-,
$R^2$ is (i) hetero ring, aryl, $C_{3-8}$ cycloalkyl or $-CO-R^0$, each of which may be substituted with group(s) selected from $-O-R^0$, $-O-R^{00}$-aryl, $-CO_2-R^0$, $-CON(-R^4)(-R^5)$, -CO-(nitrogen-containing hetero ring which may be substituted with $-R^0$), $-CONHSO_2-R^0$, -CONHOH, $-CO_2H$, $-NO_2$, and -CN, or (ii)-H, or $-R^0$,
$R^{00}$ is a bond or $C_{1-6}$ alkylene,
$R^3$ is -H, $-R^0$, $C_{1-6}$ alkyl which may be substituted with one or more halogens, halogen, $-NO_2$, -CN, or $-O-R^0$,

the dotted line is Z-olefin or E-olefin, or a mixture thereof,
provided that, (a) when $R^1$ is methoxycarbonyl, ethoxycarbonyl, N,N-dimethylaminocarbonyl or N-phenylaminocarbonyl, and $-X-R^2$ is methyl, $R^3$ represents a group other than -H, and (b) when $R^1$ is ethoxycarbonyl, $-CO_2H$ or $-CON(CH_3)_2$, and $-X-R^2$ is benzyl, $R^3$ represents a group other than -H.)
In this connection, when a symbol in a chemical formula in the present specification is also used in other chemical formulae, the same symbol has the same meaning, unless otherwise specifically described.

Further, the present invention relates to a pharmaceutical composition for preventing or treating constipation-type IBS, atonic constipation and/or functional gastrointestinal disorder containing the compound of the formula (I) or a salt thereof. In this connection, the pharmaceutical composition includes an agent for preventing or treating constipation-type IBS, atonic constipation and/or functional gastrointestinal disorder containing the compound of the formula (I) or a salt thereof.
Furthermore, the present invention relates to use of the compound of the formula (I) or a salt thereof for the manufacture of a pharmaceutical composition for preventing or treating constipation-type IBS, atonic constipation and/or functional gastrointestinal disorder; the compound of the formula (I) or a salt thereof for use in the prevention or treatment of constipation-type IBS, atonic constipation and/or functional gastrointestinal disorder; and a method for preventing or treating constipation-type IBS, atonic constipation and/or functional gastrointestinal disorder comprising administering an effective amount of the compound of the formula (I) or a salt thereof to a subject. The "subject" represents a human or other animal which needs this prevention or treatment, and in an embodiment, a human who needs this prevention or treatment.

Effects of the Invention

The compound of the formula (I) or a salt thereof has a TRPA1 channel activation activity, and can be used as an active ingredient for a pharmaceutical composition for preventing and/or treating constipation-type IBS, atonic constipation and/or functional gastrointestinal disorder, or the like.

Best Mode for Carrying Out the Invention

Hereinafter, the present invention will be described in detail.
In the present specification, the "alkyl" includes linear alkyl and branched alkyl. Accordingly, $C_{1-6}$ alkyl is linear or branched alkyl having 1 to 6 carbon atoms, specifically, for example, methyl, ethyl, n-propyl, isopropyl, n-butyl, isobutyl,

sec-butyl, tert-butyl, n-pentyl, n-hexyl, or the like. In an embodiment, it is methyl, ethyl, n-propyl, or isopropyl. In another embodiment, it is methyl or ethyl, and in another embodiment, it is methyl. $C_{4-6}$ alkyl is linear or branched alkyl having 4 to 6 carbon atoms, specifically, for example, n-butyl, n-pentyl, n-hexyl, 2-methylpropan-1-yl, 2-methylbutan-1-yl, 2,2-dimethylpropan-1-yl, 2-ethylbutan-1-yl, 3-methylbutan-1-yl, 3-methylpentan-1-yl, 2-methylpentan-1-yl, 2,2-dimethylbutan-1-yl, 2,3-dimethylbutan-1-yl, butan-2-yl, 3-methylbutan-2-yl, 3-ethylbutan-2-yl, 2-methylpentan-3-yl, or the like. In an embodiment, it is branched alkyl having 4 to 6 carbon atoms, and in another embodiment, it is 2-methylpropan-1-yl, 2-methylbutan-1-yl, 2,2-dimethylpropan-1-yl, 2-ethylbutan-1-yl, 3-methylbutan-1-yl, or 3-methylpentan-1-yl.

The "alkylene" is a divalent group where any one of hydrogen atoms of the "alkyl" is removed. Therefore, $C_{1-10}$ alkylene is linear or branched alkylene having 1 to 10 carbon atoms, specifically, for example, methylene, ethylene, trimethylene, tetramethylene, pentamethylene, hexamethylene, propan-1,2-diyl, butan-1,2-diyl, butan-1,3-dryl, butan-2,3-diyl, 2-methylpropan-1,3-diyl, 2-methylpropan-1,2-diyl, pentan-1,2-diyl, pentan-1,3-diyl, pentan-1,4-diyl, pentan-2,3-diyl, pentan-2,4-diyl, 2,2-dixnethylpropan-1,3-diyl, 2-methylbutan-1,4-diyl, 3-methylbutan-1,4-diyl, 2-ethylbutan-1,4-diyl, 3-ethylbutan-1,4-diyl, or the like. In an embodiment, it is $C_1$ alkylene, in another embodiment, it is $C_{3-8}$ alkylene, in another embodiment, it is $C_{4-6}$ alkylene, and in another embodiment, it is branched $C_{4-6}$ alkylene. Furthermore, the $C_{1-6}$ alkylene in $R^{00}$ is linear or branched alkylene having 1 to 6 carbon atoms, specifically, for example, methylene, ethylene, trimethylene, or the like, and in another embodiment, it is methylene.

The "halogen" means F, Cl, Br, or I.

The "$C_{1-6}$ alkyl which may be substituted with one or more halogens" is $C_{1-6}$ alkyl substituted with one or more halogens, which are the same or different, in addition to $C_{1-6}$ alkyl which is not substituted with halogen, specifically, for example, trifluoromethyl, fluoromethyl, difluoromethyl, 2-fluoroethyl, 3-fluoropropyl, or the like.

The "cycloalkyl" means a saturated hydrocarbon ring group. Accordingly, $C_{3-8}$ cycloalkyl is a saturated carbon ring having 3 to 8 carbon atoms, specifically, for example, cyclopropyl, cyclobutyl, cyclopentyl, cyclohexyl, cycloheptyl, or the like. In an embodiment, it is $C_{3-6}$ cycloalkyl, and in another embodiment, it is $C_{5-6}$ cycloalkyl.

The "aryl" is a $C_{6-14}$ monocyclic to tricyclic aromatic hydrocarbon ring group, and includes a partially hydrogenated ring group thereof. Specifically, for example, it is phenyl, naphthyl, tetrahydronaphthyl, indanyl, indenyl, or the like. In an embodiment, it is phenyl and naphthyl, and in another embodiment, it is phenyl.

The "hetero ring" means i) a monocyclic 3- to 8-membered ring containing 1 to 4 hetero atoms selected from oxygen, sulfur and nitrogen, and in an embodiment it is 5- to 7-membered hetero ring, and means a ring group comprising ii) a bicyclic or tricyclic hetero ring containing 1 to 5 hetero atoms selected from oxygen, sulfur and nitrogen, formed by the condensation of the monocyclic hetero ring and one or two rings selected from the group consisting of a monocyclic hetero ring, benzene ring, $C_{5-8}$ cycloalkane and $C_{5-8}$ cycloalkene. The ring atom sulfur or nitrogen may be oxidized to form oxide or dioxide.

Examples of the "hetero ring" include the following groups.

(1) Monocyclic saturated hetero ring group,

i) containing 1 to 4 nitrogen atoms, specifically, azepanyl, diazepanyl, aziridinyl, azetidinyl, pyrrolidinyl, imidazolidinyl, piperidinyl, pyrazolidinyl, piperazinyl, or the like;
ii) containing 1 to 3 nitrogen atoms, and 1 or 2 sulfur atoms and/or 1 or 2 oxygen atoms, specifically, thiomorpholinyl, thiazolidinyl, isothiazolidinyl, oxazolidinyl, morpholinyl, or the like;
iii) containing 1 or 2 sulfur atoms, specifically tetrahydrothiinyl, or the like;
iv) containing 1 or 2 sulfur atoms and 1 or 2 oxygen atoms, specifically oxathiolan, or the like; and
v) containing 1 or 2 oxygen atoms, specifically, oxiranyl, dioxolanyl, oxolanyl, tetrahydropyranyl, 1,4-dioxanyl, or the like;

(2) Monocyclic unsaturated hetero ring group,

i) containing 1 to 4 nitrogen atoms, specifically, pyrrolyl, imidazolyl, pyrazolyl, pyridyl, dihydropyridyl, pyrimidinyl, pyrazinyl, pyridazinyl, triazolyl, tetrazolyl, dihydrotriazinyl, azepinyl, or the like;
ii) containing 1 to 3 nitrogen atoms, and 1 or 2 sulfur atoms and/or 1 or 2 oxygen atoms, specifically, thiazolyl, isothiazolyl, thiadiazolyl, dihydrothiazinyl, oxazolyl, isoxazolyl, oxadiazolyl, oxazinyl, or the like;
iii) containing 1 or 2 sulfur atoms, specifically, thienyl, thiepinyl, dihydrodithiinyl, dihydrodithionyl, or the like;
iv) containing 1 or 2 sulfur atoms and 1 or 2 oxygen atoms, specifically, dihydrooxathiinyl, or the like; and
v) containing 1 or 2 oxygen atoms, specifically, furyl, pyranyl, oxepinyl, dioxolyl, or the like;

(3) Condensed polycyclic saturated hetero ring group,

i) containing 1 to 5 nitrogen atoms, specifically, quinuclidine, 7-azabicyclo[2.2.1]heptyl, 3-azabicyclo[3.2.2]nonanyl, or the like;
ii) containing 1 to 4 nitrogen atoms, and 1 to 3 sulfur atoms and/or 1 to 3 oxygen atoms, specifically, trithiadiazaindenyl dioxoloimidazolidinyl, or the like; and
iii) containing 1 to 3 sulfur atoms and/or 1 to 3 oxygen atoms, specifically, 2,6-dioxabicyclo[3.2.2]oct-7-yl, or the like;

(4) Condensed polycyclic unsaturated hetero ring group,

i) containing 1 to 5 nitrogen atoms, specifically, indolyl, isoindolyl, indolinyl, indolidinyl, benzimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, quinoxalinyl, dihydroindazolyl, benzopyrimidinyl, naphthylidinyl, quinazolinyl, cinnolinyl, or the like;
ii) containing 1 to 4 nitrogen atoms, and 1 to 3 sulfur atoms and/or 1 to 3 oxygen atoms, specifically, benzothiazolyl, dihydrobenzothiazolyl, benzothiadiazolyl, imidazothiazolyl, imidazothiadiazolyl, benzoxazolyl, benzoxadiazolyl, or the like;
iii) containing 1 to 3 sulfur atoms, specifically, benzothienyl, benzodithiinyl, or the like;
iv) containing 1 to 3 sulfur atoms and 1 to 3 oxygen atoms, specifically, benzooxathiinyl, phenoxazinyl, or the like; and
v) containing 1 to 3 oxygen atoms, specifically, benzodioxolyl, benzofuranyl, isobenzofuranyl, chromenyl, benzodihydrofuranyl, or the like.

The "nitrogen-containing hetero ring" means a ring group selected from i) and ii) of (1), i) and ii) of (2), i) and ii) of (3) and i) and ii) of (4), among the above hetero rings. In some embodiments, it is a ring group having a bond of the nitrogen atom constituting the ring.

The nitrogen-containing hetero ring in the "-CO-(nitrogen-containing hetero ring which may be substituted with $-R^0$)" of $R^1$ is in another embodiment a ring group selected from i) and ii) of (1), among the above hetero ring, and in another embodiment, it is azetidinyl, pyrrolidinyl, imidazolidinyl, piperidinyl, piperazinyl, morpholinyl, thiomorpholinyl, or the like. Furthermore, the nitrogen-containing hetero ring in the "nitrogen-containing hetero ring which may be substituted with $-R^0$" of $R^1$, that is, the nitrogen-containing hetero ring which may be substituted with $-R^0$ which is bonded to the dotted line not through -CO-, is in an embodiment a ring group selected from i) and ii) of (2) among the above hetero ring, and in another embodiment, it is imidazolyl, thiazolyl, or oxazolyl.

The nitrogen-containing hetero ring in the "-CO-(nitrogen-containing hetero ring which may be substituted with $-R^0$)" exemplified as one of the acceptable substituents on the hetero ring, aryl, $C_{3-8}$ cycloalkyl or $-CO-R^0$ of $R^2$, is in an embodiment a ring group selected from i) and ii) of (1) among the above hetero ring, and in another embodiment, it is azetidinyl, pyrrolidinyl, imidazolidinyl, piperidinyl, morpholinyl, or thiomorpholinyl. Furthermore, the hetero ring in the "hetero ring, aryl, $C_{3-8}$ cycloalkyl or $-CO-R^0$, each of which may be substituted" of $R^2$ is in an embodiment a ring group selected from i) to v) of (2) among the above hetero ring, specifically, for example, pyridyl, imidazolyl, thiazolyl, oxathiazolyl, pyrazyl, pyrimidinyl, or pyridazinyl, and in another embodiment, it is pyridyl.

The "cyclic amino" is i) and ii) of (1) among the above "nitro-containing hetero ring", and is a group having a bond at the nitrogen atom constituting the ring. Specifically, it is, for example, pyrrolidin-1-yl, azetidin-1-yl, morpholin-4-yl, piperazin-1-yl, piperidin-1-yl, thiomorpholin-4-yl, azepan-1-yl, 1,4-diazepan-1-yl, or the like. In another embodiment, it is pyrrolidin-1-y1, azetidin-1-yl, morpholin-4-yl, or piperazin-1-yl.

The "$-CO_2H$ or a biological equivalent thereof" means $-CO_2H$ or, other atoms or atomic groups which has the same electrical or steric arrangement as $-CO_2H$, and has common biological properties capable of discharging acidic proton. For example, $-CO_2H$, hydroxamic acid ($-CO-NH-OH$, $-CO-NH-O-R^0$), sulfonamide ($-NH-SO_2-R^0$), acyl cyanamide ($-CO-NH-CN$), acyl sulfonamide ($-CO-NH-SO_2-R^0$), $-SO_2-NH-CO-R^0$, or tetrazolyl, oxadiazolonyl, oxadiazol thionyl, oxathiadiazolyl, thiadiazolonyl, triazole thionyl, hydroxyisoxazolyl, or the like, in another embodiment, it is $-CO_2H$, acyl sulfonamide ($-CO-NH-SO_2-R^0$) or hydroxamic acid ($-CO-NH-OH$, $-CO-NN-O-R^0$), and in another embodiment, it is $-CO_2H$.

The dotted line means E-olefin or Z-olefin represented by the following formula E-(I) or Z-(I), or a mixture of these. In an embodiment, it is E-olefin or a mixture of E-olefin and Z-olefin, and in another embodiment, it is E-olefin.

[chem. 13]

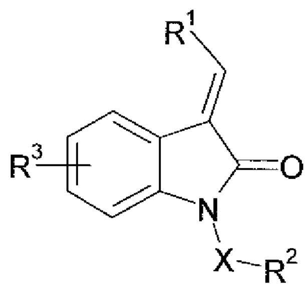

E-(I)

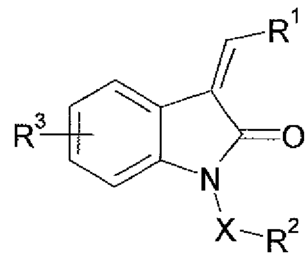

Z-(I)

In the present specification, the "which may be substituted" means unsubstituted, or substituted with the same or different, 1 to 5 substituent(s). In this connection, when a plurality of substituents are present, these substituents may be the same as or different from each other.

Embodiments of the compound of the formula (I) or a salt thereof are shown below.

(1) A compound or a salt thereof, wherein the dotted line is E-olefin.

(2) A compound or a salt thereof, wherein $R^1$ is $-CO_2H$, $-CONH_2$, $-CON(CH_3)_2$, or -CO-(cyclic amino which may be substituted with $-R^0$). In another embodiment, a compound or a salt thereof, wherein $R^1$ is $-CO_2H$, $-CONH_2$, $-CON(CH_3)_2$, or pyrrolidin-1-ylcarbonyl, azetidin-1-ylcarbonyl, or morpholin-4-ylcarbonyl.

(3) A compound or a salt thereof, wherein $-X-R^2$ is $C_{4-6}$ alkyl. In another embodiment, a compound or a salt thereof, wherein $-X-R^2$ is 2-methylpropan-1-yl, 2-methylbutan-1-yl, 2,2-dimethylpropan-1-yl, 2-ethylbutan-1-yl, 3-methylbutan-1-yl or 3-methylpentan-1-yl. In another embodiment, a compound or a salt thereof, wherein $-X-R^2$ is cyclopropylmethyl, cyclobutylmethyl, cyclopentylmethyl, or cyclohexylmethyl. In another embodiment, a compound or a salt thereof which is benzyl which may have substituent(s) selected from the group consisting of $-O-R^0$ and $-CO_2-R^0$ on the benzene ring. In another embodiment, a compound or a salt thereof, wherein $-X-R^2$ is benzyl.

(4) A compound or a salt thereof, wherein $R^3$ is -H, -F or -Cl. In another embodiment, a compound or a salt thereof, wherein $R^3$ is -F. In another embodiment, a compound or a salt thereof, wherein $R^3$ is 7-fluoro. In another embodiment, a compound or a salt thereof, wherein $R^3$ is 5-fluoro. In another embodiment, a compound or a salt thereof, wherein $R^3$ is -H. In another embodiment, a compound or a salt thereof, wherein $R^3$ is 7-chloro.

(5) A compound or a salt thereof which is a combination of two or more groups among the groups described in (1) to (4) above.

The present invention includes a compound or a salt thereof which is a combination of two or more groups among the groups described in (1) to (4) above, as described in (5) above, and specific examples thereof include the following embodiments.

(6) The compound of the formula (I) or a salt thereof, wherein $R^1$ is $-CO_2H$ or a biological equivalent thereof, $-CO_2-R^0$, $-CON(-R^4)(-R^5)$, -CN, -CO-(nitrogen-containing hetero ring), or nitrogen-containing hetero ring which may be substituted with $-R^0$, $R^4$ and $R^5$ are the same or different, representing -H or $C_{1-6}$ alkyl, and $R^2$ is (i) hetero ring, aryl, cycloalkyl or $-CO-R^0$, each of which may be substituted with group(s) selected from $-OR^0$, $-O-R^{00}$-aryl, $-CO_2-R^0$, $-CON(-R^4)(-R^5)$, -CO-(nitrogen-containing hetero ring), $-CONHSO_2-R^0$, -CONHOH, $-NO_2$ and -CN, or (ii) -H or $-R^0$.

(7) The compound of the formula (I) or a salt thereof, wherein $R^1$ is $-CO_2H$, $-CON(-R^4)(-R^5)$, -CN, -CO-(nitrogen-containing hetero ring which may be substituted with $-R^0$), or nitrogen-containing hetero ring which may be substituted with $-R^0$, $R^2$ is (i) hetero ring, aryl or cycloalkyl, each of which may be substituted with group(s) selected from $-O-R^0$, $-O-R^{00}$-aryl, $-CO_2-R^0$ and $-CO_2H$, or (ii) -H, and $R^3$ is -H, $-R^0$, halogen or $-O-R^0$.

(8) The compound or a salt thereof of (7), wherein the dotted line is E-olefin.

(9) The compound or a salt thereof of (8), wherein $R^1$ is $-CO_2H$, $-CONH_2$, $-CON(CH_3)_2$ or -CO-(cyclic amino which may substituted with $-R^0$).

(10) The compound or a salt thereof of (9), wherein $R^3$ is -H, -F or -Cl.

(11) The compound or a salt thereof of (10), wherein $-X-R^2$ is $C_{4-6}$ alkyl.

(12) The compound or a salt thereof of (11), wherein $-X-R^2$ is 2-methylpropan-1-yl, 2-methylbutan-1-yl, 2,2-dimethylpxopan-1-yl, 2-ethylbutan-1-yl, 3-methylbutan-1-yl or 3-methylpentan-1-yl.

(13) The compound or a salt thereof of (10), wherein $-X-R^2$ is $C_{3-8}$ cycloalkylmethyl or benzyl in which the benzene ring may be substituted with group(s) selected from the group consisting of $-O-R^0$ and $-CO_2-R^0$.

(14) The compound or a salt thereof of (13), wherein $-X-R^2$ is cyclopropylmethyl, cyclobutylmethyl, cyclopentyl-

methyl, cyclohexylmethyl or benzyl.

(15) The compound or a salt thereof of (12) or (14), wherein $R^1$ is $-CO_2H$.

(16) The compound or a salt thereof of (12) or (14), wherein $R^1$ is $-CONH_2$ or $-CON(CH_3)_2$.

(17) The compound or a salt thereof of (12) or (14), wherein $R^1$ is pyrrolidin-1-ylcarbonyl, azetidin-1-ylcarbonyl or morpholin-4-ylcarbonyl.

Furthermore, as specific examples included in the compound of the formula (I) or a salt thereof, the following compounds can be exemplified;

(2E)-[1-(cyclohexylmethyl)-2-oxo-1,2-dihydro-3H-indol-3-ylidene]acetic acid,
(2E)-(1-benzyl-5-fluoro-2-oxo-1,2-dihydro-3H-indol-3-ylidene)acetic acid,
(2E)-(1-benzyl-7-fluoro-2-oxo-1,2-dihydro-3H-indol-3-ylidene)acetic acid,
(2E)-[1-(cyclopentylmethyl)-2-oxo-1,2-dihydro-3H-indol-3-ylidene]acetic acid,
(2E)-(7-fluoro-1-isobutyl-2-oxo-1,2-dihydro-3H-indol-3-ylidene)acetic acid,
(2E)-[1-(cyclopentylmethyl)-7-fluoro-2-oxo-1,2-dihydro-3H-indol-3-ylidene] acetic acid,
(2E)-(7-chloro-1-isobutyl-2-oxo-1,2-dihydro-3H-indol-3-ylidene)acetie acid,
(2E)-[1-(cylobutylmethyl)-7-fluoro-2-oxo-1,2-dihydro-3H-indol-3-ylidene] acetic acid,
(2E)-[1-(cylopropylmethyl)-7-fluoro-2-oxo-1,2-dihydro-3H-indol-3-ylidene] acetic acid,
(2E)-2-[1-(cyclopentylmethyl)-7-fluoro-2-oxo-1,2-dihydro-3H-indol-3-ylidene]-N,N-dimethylacetamide,
(3E)-1-(2-ethylbutyl)-7-fluoro-3-(2-morpholin-4-yl-2-oxoethylidene)-1,3-dihydro-2H-indol-2-one,
(2E)-{7-fluoro-1-[(2S)-2-methylbutyl]-2-oxo-1,2-dihydro-3H-indol-3-ylidene}acetic acid,
(2E)-[7-fluoro-1-(3-methylbutyl)-2-oxo-1,2-dihydro-3H-indol-3-ylidene] acetic acid,
(2E)-2-[1-(cyclohexylmethyl)-2-oxo-1,2-dihydro-3H-indol-3-ylidene]-N,N-dimethylacetamide,
(2E)-2-[1-(cyclopentylmethyl)-2-oxo-1,2-dihydro-3H-indol-3-ylidene]-N,N-dimethylacetamide,
(3E)-3-(2-azetidin-1-yl-2-oxoethylidene)-1-(cyclohexylmethyl)-1,3-dihydro-2H-indol-2-one,
(3E)-3-(2-azetidin-1-yl-2-oxoethylidene)-1-(cyclopentylmethyl)-1,3-dihydro-2H-indol-2-one,
(2E)-[1-(2-ethylbutyl)-2-oxo-1,2-dihydro-3H-indol-3-ylidene]acetic acid,
(3E)-1-(2-ethylbutyl)-3-(2-oxo-2-pyrrolidin-1-ylethylidene)-1,3-dihydro-2H-indol-2-one,
(3E)-3-(2-azetidin-1-yl-2-oxoethylidene)-1-(2-ethylbutyl)-1,3-dihydro-2H-indol-2-one,
(3E)-3-(2-azetidin-1-yl-2-oxoethylidene)-1-(cyclobutylmethyl)-1,3-dihydro-2H-indol-2-one, or,
(3E)-1-(cyclobutylmethyl)-3-(2-oxo-2-pyrrolidin-1-ylethylidene)-1,3-dihydro-2H-indol-2-one,

and a salt thereof.

The compound of the formula (I) or a salt thereof may in some cases exist in tautomers or geometrical isomers, depending on the kinds of the substituents. In the present specification, the compound of the formula (I) or a salt thereof may be described in only one form of isomers, but the present invention includes other isomers, isolated forms of the isomers, or a mixture thereof.

Furthermore, the compound of the formula (I) or a salt thereof may have asymmetric carbon atoms or asymmetries in some cases, and correspondingly, it may exist in optical isomers. The present invention includes the isolated form of the optical isomer of the compound of the formula (I) or a salt thereof or a mixture thereof.

Additionally, pharmaceutically acceptable prodrugs of the compound of the formula (I) are also included in the present invention. The pharmaceutically acceptable prodrug refers to a compound having a group which can be converted into an amino group, a hydroxyl group, a carboxyl group, or the like, by solvolysis or under a physiological condition. Examples of the groups forming a prodrug include those as described in, for example, Prog. Med., 5, 2157-2161 (1985) or "Pharmaceutical Research and Development" (Hirokawa Publishing Company, 1990), vol. 7, Drug Design, 163-198.

Furthermore, the salt of the compound of the formula (I) is a pharmaceutically acceptable salt of the compound of the formula (I), and some of the compounds of the formula (I) may form an acid addition salt or a salt with a base, depending on the kinds of the substituents. Specifically, examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditolyl tartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like, and with organic bases such as methylamine, ethylamine, ethanolamine, lysine, ornithine, and the like, salts with various amino acids such as acetyl leucine and the like or derivatives of amino acids, ammonium salts, and others.

Additionally, the present invention also includes various hydrates or solvates, and polymorphism of the compound of the formula (I) and a salt thereof. Furthermore, the present invention also includes the compounds labeled with various radioactive or nonradioactive isotopes.

(Production Processes)

The compound of the formula (I) or a salt thereof can be prepared by applying various known synthetic methods, using the characteristics based on their basic structures or the kinds of the substituents. At this time, depending on the types of the functional groups, it is in some cases effective from the viewpoint of the preparation techniques to protect the functional group with an appropriate protecting group (a group which is capable of being easily converted into the functional group), during the steps from starting materials to intermediates. Examples of the protecting group include the protective groups as described in "Green's Protective Groups in Organic Synthesis (4th edition, 2006)", edited by P. G. M. Wuts and T. W. Greene, and the like, which may be appropriately selected and used depending on the reaction conditions. In these methods, a desired compound can be obtained by introducing the protecting group to carry out the reaction, and then, if desired, removing the protecting group.
Additionally, the prodrug of the compound of the formula (I) or a salt thereof can be prepared by introducing a specific group during the steps from starting materials to intermediates, in the same manner as for the above protecting groups, or by further carrying out the reaction using the obtained compound of the formula (I) or a salt thereof. The reaction can be carried out by applying a method known by a person skilled in the art, such as general esterification, amidation, dehydration, and the like.
Hereinbelow, typical production processes of the compound of the formula (I) will be described. Each of the production processes can also be carried out with reference to the documents appended to the description herein. In this connection, the production process of the compound of the formula (I) is not limited to the examples as shown below.

(Production Process 1)

[chem. 14]

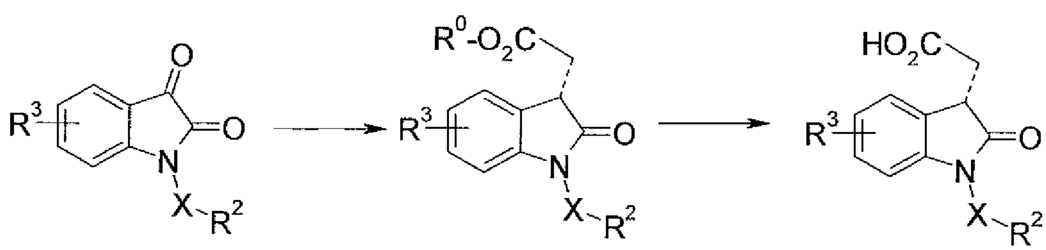

(II) (I-a) (I-b)

The production process is a method where olefin is introduced to a compound (II), and then the obtained compound (I-a) is hydrolyzed to thereby produce a compound (I-b) as the compound of the present invention. The compound (I-a) can be produced by reacting various carboanions to the compound (II). Examples of the carboanions include carboanions stabilized by adjacent hetero atom such as phosphorus ylide and the like, phosphoryl group-substituted carboanion, α-silyl carboanion, or similar types of chemicals thereof.
Furthermore, the compound (I-a) can be produced by the reaction of the compound (II) and corresponding phosphorus ylide. In this, the compound (II) and phosphorus ylide are used in an equivalent molar or in an excess amount of either thereof, and a mixture of these is stirred in a solvent inert to the reaction, under cooling to heating with reflux, for example, at 0˚C to 80˚C, usually for 0.1 hour to 5 days. Examples of the solvent to be used herein are not specifically limited, but include aromatic hydrocarbons such as benzene, toluene, and xylene, ethers such as diethylether, tetrahydrofuran (THF), dioxane, and dimethoxyethane (DME), halogenated hydrocarbons such as dichloromethane (DCM), 1,2-dichloroethane (DCE), and chloroform, and a mixture thereof.
Furthermore, the compound (I-b) can be produced by hydrolyzing the ester moiety of the obtained compound (I-a).

(Production Process 2)

[chem. 15]

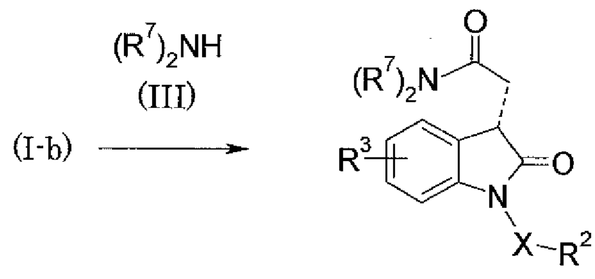

(I-c)

(In the formula, $-N(R^7)_2$ represents $-N(-R^4)(-R^5)$ or a nitrogen-containing hetero ring which may be substituted with $-R^0$.) The production process is a method where the compound (I-c) as the compound of the present invention is produced by amidation reaction of the compound (I-b) and a compound (III). The reaction is carried out, using the compound (I-b) and the compound (III) in an equivalent molar or in excess amount of either thereof are used, by stirring in a solvent inert to the reaction in the presence of a condensation agent, under cooling to heating, for example, at -20˚C to 60˚C, usually for 0.1 hour to 5 days. The reaction solvent is not specifically limited, but examples thereof include aromatic hydrocarbons, halogenated hydrocarbons, ethers, N,N-dimethylformamide (DMF), dimethyl sulfoxide (DMSO), ethyl acetate, acetonitrile or water, or a mixture thereof. As a condensation agent, it is in some cases preferable to use 1-[bis(dimethylamino)methylene]-1H-1,2,3-triazolo[4,5-b]pyridin-1-ium-3-oxide hexafluorophosphate (HATU), 1-(3-dimethylaminopropyl)-3-ethyl carbodiimide hydrochloride (EDCI·HCl), dicyclohexyl carbodiimide (DCC), 1,1'-carbonyldiimidazol (CDI), diphenyl azide phosphate, phosphorus oxychloride, or a polystyrene resin holding a condensation agent, for example, PS-carbodiimide (Argonaut Technologies, US) or the like, but it is not limited thereto. It is in some cases preferable to use additives (for example, 1-hydroxybenzotriazole (HOBt), or the like) in the reaction, for example, it is advantageous for the smooth reaction to react in the presence of an organic base such as triethylamine, N,N-diisopropylethylamine (DIPEA) or N-methyl morpholine, or an inorganic base such as potassium carbonate, sodium carbonate or potassium hydroxide. For the purpose of removing the excess amine after the completion of the reaction, a polystyrene resin holding isocyanate, for example, PS-isocyanate (Argonaut Technologies, US) or the like may be used. Furthermore, for the purpose of removing the excess carboxylic acid, the above-described additive or the like after the completion of the reaction, a polystyrene resin holding quaternary ammonium salt, for example, MP-carbonate (Argonaut Technologies, US) or the like may be used.
Furthermore, a method where the compound (I-b) is introduced into a reactive derivative, and then reacted with the compound (III), may be used. Herein, as the reactive derivative of the compound (I-b), an acid halide obtained by the reaction with halogenating agents such as phosphorus oxychloride, thionyl chloride, mixed acid anhydride obtained by the reaction with isobutyl chloroformate or the like, activated ester obtained by the condensation with HOBt or the like, are exemplified. The reaction of these reactive derivatives and the compound (III) can be performed in a solvent inert to the reaction such as halogenated hydrocarbons, aromatic hydrocarbons, ethers, or the like, under cooling to heating, for example at -20˚C to 60˚C.
The compound of the formula (I) as the compound of the present invention can be produced by various functional group conversions, such as deprotection, acylation, akylation, sulfonylation, from the obtained compound (I-c) as the compound of the present invention.

(Production Process 3)

[chem. 16]

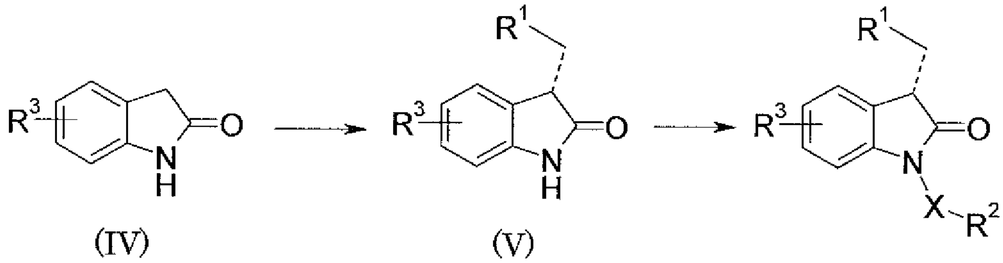

(IV) (V) (I-d)

The production process is a method where a compound (IV) and corresponding aldehyde are reacted in the presence of a base, to produce a compound (V), and then the compound (I-d) as the compound of the present invention is produced by N-alkylation.

In production of the compound (V), the compound (IV) and aldehyde are used in a equivalent molar or in excess amount of either thereof, and a mixture of these is stirred in a solvent inert to the reaction, at -45°C to heating with reflux, for example at 0°C to heating with reflux, usually for 0.1 hour to 5 days. Examples of the solvent to be used herein are not specifically limited, but include alcohols such as methanol (MeOH), ethanol (EtOH), ethers, and mixtures thereof. As the base, for example, catalytic amount of a base such as piperidine may be used, and various additives for promoting the reaction may be used.

(Starting Material Synthesis)

Starting Material Production Process 1

[chem. 17]

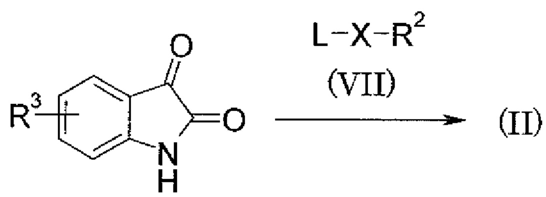


(VI)

(In the formula, L represents a leaving group, for example, halogen, $-SO_2-C_{1-6}$ alkyl which may be substituted with one or more halogen(s), or benzenesulfonyloxy which may be substituted with group(s) selected from the group consisting of $-R^0$, halogen, and nitro.)

The compound (II) can be produced by an alkylation reaction of compound (VI) and a compound (VII). The reaction is performed by stirring in a solvent inert to the reaction in the presence of a base, under cooling to heating with reflux, for example, at 0°C to 80°C, usually for 0.1 hour to 5 days. The solvent is not specifically limited, but aromatic hydrocarbons, ethers, halogenated hydrocarbons, DMF, DMSO, ethyl acetate, acetonitrile or a mixture of these are exemplified. The base includes organic bases such as triethylamine (TEA), DIPEA, 1,8-diazabicydo[5.4.0]-7-undecene (DBU), and n-butyl lithium, and inorganic bases such as sodium carbonate, potassium carbonate, sodium hydride, and potassium tert-butoxide. Furthermore, the present reaction can be performed in the presence of a phase transfer catalyst such as tetra-n-butyl ammonium chloride or copper-copper iodide catalyst, or the like.

The compound of the formula (I) is isolated and purified as their free compounds, salts thereof, hydrates,

solvates, or polymorphic substances. The salt of the compound of the formula (I) can be prepared by subjecting to a conventional salt formation reaction.
Isolation and purification can be carried out by employing general chemical operations such as extraction, fractional crystallization, various types of fractional chromatography, and the like.
Various isomers can be prepared by selecting a suitable starting compound or separated by making use of the difference in the physicochemical properties among the isomers. For example, the optical isomers can be obtained by means of general optical resolution methods of racemic compounds (for example, by fractional crystallization introducing the compound into diastereomer salts with optically active bases or acids, chromatography using a chiral column or the like, and others), or can also be prepared from a suitable optically active starting compound.

The pharmacological activity of the compound of the formula (I) or a salt thereof was confirmed by the following tests.

Test Example 1 Isolation of TRPA1 gene derived from human and construction of the expression vector

10 ng of Human brain mRNA (Clontech Co.) was treated with DNase, and then was reverse transcribed using kit for reverse transcription-polymerase chain reaction (RT-PCR) (SUPERSCRIPT First-Strand Synthesis System for RT-PCR; Invitrogen Co.) to synthesize the first strand cDNA. PCR using a hot start method was performed using the first strand cDNA as a template and Taq DNA polymerase (LA Taq DNA polymerase; TAKARA SHUZO). The PCR was performed using oligonucleotides consisting of the nucleotide sequences, represented by sequence number 2 as a sense primer and sequence number 3 as an antisense primer, in which firstly, heat denaturation was performed at 98˚C (1 minute), and then a cycle of 98˚C (15 seconds)/56˚C (30 seconds)/72˚C (5 minutes) was repeated 35 times. As a result, a DNA fragment of about 3.3kbp was amplified.
The DNA fragment was cloned to pCR-TOPO vector using a cloning kit (TOPO XL PCR Cloning Kit; Invitrogen). The obtained plasmid DNA was digested with restriction enzymes KpnI and HindIII, and then was cloned using plasmid pcDNA 3.1 (+)(Invitrogen). In this connection, the plasmid pcDNA 3.1 (+) has a promoter sequence derived from cytemegalovirus, and can be used in order to express a protein in animal cells.
The nucleotide sequences of the obtained clones were analyzed using DNA sequencer (ABI3700 DNA Sequencer; Applied Biosystems) by dideoxyterminator method, and the nucleotide sequence represented by sequence number 1 was obtained. Furthermore, when these sequences were translated to amino acid sequences, the amino acid sequence represented by sequence number 1 was obtained.

Test Example 2 Measurement of TRPA1 activation activity

After constructing the expression vector of the human TRPA1 gene, selection by G418 was performed from the HEK293 cells which temporarily expressed TRPA1 by transfection operation to obtain cells stably expressing TRPA1. The above operation was performed according to known methods (experimental medicine supplementary volume, "Cultivation Cell Experimental Methods for Molecular Biology Study" published by Toshio Kuroki, Namho Hue and Kazuhiro Chida, 1995, Yodosha).
In order to measure the change of the intracellular calcium concentration in the cells stably expressing the human TRPA1, the change of the intracellular calcium concentration when a compound to be tested was added was measured using FLIPR (Molecular Device).
In order to measure the change of the intracellular calcium concentration with FLIPR, the following pretreatment was performed. First of all, assay buffer was prepared which was for adding fluorescent pigment Fluo4-AM (DOJIN) to the cells or washing the cells just before performing FLIPR assay. To a solution (hereinafter, HBSS/HEPES solution) where 20 mL of 1M HEPES (pH 7.4; Invitrogen) was added to 1000 mL of a physiological saline buffer (Hank's Balanced Salt Solution; HBSS) (Invitrogen), was added 10 mL of a solution where 710 mg of probenecid (Sigma) was dissolved into 5 mL of 1M aqueous NaOH solution and 5 mL of HBSS/HEPES solution was further added and mixed, and the assay buffer was thus prepared. Then, 50 $\mu$g of Fluo4-AM was dissolved into 22 $\mu$L of DMSO (DOJIN), the same amount of 20% pluronic acid (Molecular Probes) was added thereto and mixed, and the mixture was added to the 10.6 mL of the assay buffer where 105 $\mu$L of fetal bovine serum was added, to prepare a fluorescent pigment solution. The medium of the cells stably expressing TRPA1 was removed, 100 $\mu$L per well of the fluorescent pigment solution was dispensed immediately, followed by cultivation in a $CO_2$ cultivator for 1 hour, and the fluorescent pigment was soaked in the cells. The cells after cultivation were washed using the assay buffer, followed by being set in FLIPR. Furthermore, a test sample, which was added to the cells stably expressing TRPA1, was prepared by using the assay buffer, and at the same time, was set in FLIPR. The above-mentioned pretreatment was performed, and then the change of the intracellular calcium concentration after the addition of the compound to be tested was measured with FLIPR, $EC_{50}$ value was calculated according to a conventional method.
$EC_{50}$ values of some compounds of the formula (I) are shown in Table 1

[Table 1]

| Ex | $EC_{50}$ [μM] | Ex | $EC_{50}$ [μM] | Ex | $EC_{50}$ [μM] |
|---|---|---|---|---|---|
| 1 | 4.27 | 27 | 4.63 | 43 | 3.76 |
| 4 | 1.20 | 28 | 0.51 | 45 | 1.29 |
| 15 | 4.40 | 29 | 0.66 | 47 | 1.01 |
| 17 | 4.45 | 31 | 0.61 | 49 | 0.83 |
| 21 | 5.65 | 32 | 1.16 | | |
| 26 | 3.00 | 33 | 1.30 | | |

Test Example 3 Measurement of 5-HT secretion from RIN14B

In order to investigate whether TRPA1 is related to 5-HT release, 5-HT secretion promotion from RIN14B by a compound to be tested was measured.
The RIN14B cells cultivated in a petri dish were scraped using Phosphate buffered saline (PBS) containing 1mM EDTA, distributed into 24 well plates, and cultivated for 2 days. As a medium, a medium in which 10% fetal bovine serum (ICN), 100 U/mL penicillin, and 100 μg/mL streptomycin was added to RPMI1640 (Invitrogen), was used. The cells were washed once with HBSS (Invitrogen) and added with 0.1% BSA and 10 μM fluoxetine (TOCRIS Co.), followed by dilution with the above HBSS, the prepared compound to be tested was added to RIN14B cells, followed by cultivation at 37˚C, under 5% $CO_2$ condition for 20 minutes. After cultivation, supernatant of cells was recovered, followed by freeze preservation. The content of 5-HT in the supernatant was measured by a commercial serotonin immunoassay kit (Beckman). 5-HT release promotion activation value (%) was evaluated as a relative value when the 5-HT release promotion value by 300 μM of allyl isothiocyanate was defined as 100%, and the 5-HT release promotion value by HBSS for dilution only was defined as 0%.
As a result, the compound of the formula (I) showed a 5-HT release promotion activity, for example, the 5-HT release promotion activation values of the compounds of Example 4, Example 15, Example 21, Example 26, and Example 27 were 144.6%, 97.4%, 41.2%, 102.5%, 40.0%, respectively.

Test example 4 Activity to gastric emptying of TRPA1 agonist

When EC cell is stimulated by content of a gastrointestinal tract, 5-HT is secreted. The secreted 5-HT promotes the secretion or motility through a 5-HT receptor present in intestinal nerve plexus or gastrointestinal smooth muscle. The secreted 5-HT is instantly taken into a cell by its specific transporter, and is metabolized by monoamine reductase. Thus, while it is considerably difficult to directly measure the secreted 5-HT concentration, it has been known that gastric emptying is delayed as a physiological activity through 5-HT in rodents. In order to verify in vivo activity through 5-HT of the compound to be tested, activity to gastric emptying of rats by various TRPA1 agonists was studied.
In the experiment, male wistar rats (body weight 290 to 360g) were used. The animals were purchased from Japan SLC (Shizuoka). The animals were allowed to drink water freely under a constitutive environment.
5 minutes after the medicine was orally administered, 0.05% phenol red solution (1.5 mL) was orally administered. After 15 minutes, rats were put painlessly to death by cervical vertebral dislocation, their stomachs were exenterated, and phenol red was eluted with 0.1M aqueous NaOH solution (5 mL) from the exenterated stomachs. 20% aqueous trichloroacetic acid solution (0.1 mL) was added to the NaOH solution (0.5 mL), followed by stirring and centrifugation at 15,000 rpm, at 4˚C, for 10 minutes. 0.5M aqueous NaOH solution (0.2 mL) was added to the supernatant (0.05 mL), followed by stirring, and absorbance at 560 nm was measured using an absorption spectrometer (spectrometer spectramax M2; Molecular Devices, CA, USA).
A gastric emptying was calculated by the following calculating formula.

$$\text{Gastric emptying (\%)} = 100 - (A/B) \times 100$$

A: amount of phenol red remaining in stomach
B: amount of phenol red remaining in stomach just after administering phenol red

$ED_{50}$ values (amount of the compound to be tested which suppresses 50% of gastric emptying, with respect to the control animals without administering the compound to be tested) of some of the compounds of the formula (I) are shown in Table 2. In this connection, comparative compound 1 and comparative compound 2 are (3E)-1-methyl-3-(2-oxopropyridene)-1,3-dihydro-2H-indol-2-one, and (3E)-3-(2-oxopropyridene)-1-prop-2-in-1-yl-1,3-dihydro-2H-indol-2-one described in Non Patent Document 4 above, and has the following chemical structures, respectively.

[chem. 18]

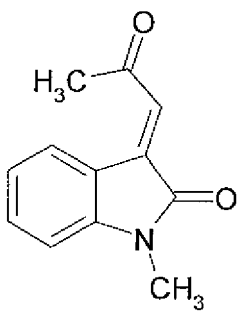

Comparative Compound 1

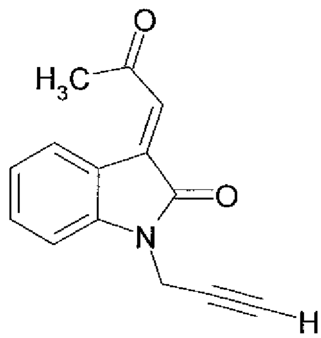

Comparative Compound 2

[Table 2]

| Ex | $ED_{50}$ [mg/kg] | Ex | $ED_{50}$ [mg/kg] |
|---|---|---|---|
| 1 | 0.13 | 32 | 0.21 |
| 13 | 0.98 | 36 | 0.069 |
| 17 | 0.31 | 39 | 0.24 |
| 18 | 3.6 | 40 | 0.59 |
| 20 | 4.4 | 45 | 0.57 |
| 24 | 0.53 | 47 | 0.61 |
| 25 | 0.31 | comparative compound 1 | 2.8 |
| 28 | 0.44 | comparative compound 2 | 8.8 |
| 30 | 0.42 | | |

Test Example 5 Loperamide induced constipation model

It has been known that loperamide as a μl opioid receptor agonist causes convulsive contraction in intestinal tracts. Thus, a delay in the intestinal transport ability due to loperamide is thought as a constipation IBS model.
Mice (Slc:ddY, five-week old, male) fasted from the previous evening of the experiment. On the day of the experiment, the mice were acclimatized to cages for measurement for 1 hour or more, and 0.3 mg/kg of loperamide was administered subcutaneously. After 30 minutes, a compound to be tested was administered orally, ether anesthesia was applied to the mice immediately thereafter, glass beads having diameter of 3 mm were inserted to 2 cm from anus. The mice were returned to the cages for measurement, and the time from waking to discharging of the glass beads was measured. Furthermore, the compound to be tested was dissolved into 10% DMSO, 10% cremophor, and 80% distillated water.
As a result, it has been recognized that there was a delay in the beads discharging time of the loperamide administration group (vehicle group) in comparison with the loperamide non-administration group (control group).
$ED_{50}$ values (amount of the compound to be tested which suppresses 50% of the delay of discharging time induced by loperamide) of some of the compounds of the formula (I) are shown in Table 3. In this connection, comparative compound 1 is the same compound as comparative compound 1 in Test Example 4.

[Table 3]

| Ex | $ED_{50}$ [mg/kg] |
|---|---|
| 13 | 0.4 |
| 24 | 0.27 |
| 26 | 0.51 |
| 28 | 0.10 |
| 31 | 0.24 |
| comparative compound 1 | 2.5 |

Test Example 6 Cytochrome P450(2D6) enzyme inhibition test

The experiment was performed in accordance with the method of Crespi et al. (Analytical Biochemistry, 248, 188-190, 1997).
As a substrate, 3-[2-(N,N-diethyl-N-methylamino)ethyl]-7-methoxy-4-methyl coumarine (1.5 μmol/L) a compound to be tested ($3.1\times10^{-7}$ to 2x $10^{-5}$ M) and an enzyme ($2.5\times10^{-8}$ M) were incubated at 37˚C for 20 minutes in total of 200 mL of 100 mM phosphorous buffer (pH=7.4) including 8.2 mM NADP+, 0.41 mM glucose-6-phosphate, 0.41 mM $MgCl_2$ and 0.4 Units/mL glucose-6-phosphate dehydrogenase , using 96 well plates. Then, 0.5 M aqueous 2-amino-2-hydroxymethyl-1,3-propandiol solution containing 80% acetonitrile was added to stop the reaction, fluorescence intensity (excitation wavelength; 390nm, fluorescent wavelength; 460 nm) was measured with a fluorescent plate reader. An inhibition ratio was calculated with the following formula, and the test compound concentration when the inhibition ratio is 50% ($IC_{50}$) was obtained. The result is shown in Table 4.

$$\text{Inhibition ratio } (\%) = 100 - (C_1 - B_1)/(C_0 - B_1) \times 100$$

$C_1$: fluorescence intensity in the presence of the test compound and enzyme at know concentrations, and the substrate
$C_0$: fluorescence intensity in the presence of the enzyme and substrate, without the test compound
$B_1$: fluorescence intensity of blank well

[Table 4]

| Ex | IC50 (μM) | Ex | IC50 (μM) |
|---|---|---|---|
| 1 | >20 | 32 | >50 |
| 15 | >20 | 37 | >50 |
| 17 | >20 | 43 | >25 |
| 26 | >20 | 45 | 18 |
| 28 | >25 | | |

From the results of the tests above, it was confirmed that the compound of the formula (I) or a salt thereof has TRPA1 channel activation activity, and can be used as an active ingredient for a pharmaceutical composition for preventing or treating constipation-type IBS, atonic constipation and/or functional gastrointestinal disorder.
A pharmaceutical composition containing one or two or more kinds of the compound of the formula (I) or a salt thereof as an active ingredient can be prepared in accordance with a generally used method, using an excipient usually used in the art, that is, a pharmaceutical excipient, a pharmaceutical carrier, or the like.
The administration can be carried out in any form of oral administration via tablets, pills, capsules, granules, powders, liquid preparations, or the like; or parenteral administration via injections such as intraarticular, intravenous, or intramuscular injections, suppositories, eye drops, eye ointments, percutaneous liquid preparations, ointments, percutaneous patches, transmucosal liquid preparations, transmucosal patches, inhalations, and the like.

As the solid composition for oral administration, tablets, powders, granules, or the like are used. In such a solid composition, one or more kinds of active ingredients are mixed with at least one inert excipient. According to a conventional method, the composition may contain inert additives such as a lubricant, a disintegrator, a stabilizing agent, and a solubilizing agent. As occasion demands, the tablets or the pills may be coated with a sugar coating, or a film of a gastric or enteric material.

The liquid composition for oral administration includes pharmaceutically acceptable emulsions, solutions, suspensions, syrups, elixirs, or the like, and contains a generally used inert diluent such as purified water or ethanol. In addition to the inert diluent, this liquid composition may contain an auxiliary agent such as a solubilizing agent, a moistening agent, and a suspending agent, a sweetener, a flavor, an aroma, and an antiseptic.

The injections for parenteral administration include sterile aqueous or non-aqueous liquid preparations, suspensions and emulsions. The aqueous solvent includes, for example, distilled water for injection and physiological saline. Examples of the non-aqueous solvent include alcohols such as ethanol. Such a composition may further contain a tonicity agent, an antiseptic, a moistening agent, an emulsifying agent, a dispersing agent, a stabilizing agent, or a solubilizing agent. These are sterilized, for example, by filtration through a bacteria retaining filter, blending of a bactericide, or irradiation. Additionally, these can also be used by preparing a sterile solid composition, and dissolving or suspending it in sterile water or a sterile solvent for injection prior to its use.

The agent for external use includes ointments, plasters, creams, jellies, cataplasms, sprays, lotions, eye drops, eye ointments, and the like. The agents contain generally used ointment bases, lotion bases, aqueous or non-aqueous liquid preparations, suspensions, emulsions, and the like.

As the transmucosal agents such as an inhalation, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate propellant, for example, a suitable gas such as chlorofluoroalkane, carbon dioxide, and the like, or other forms.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. Additionally, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

Although it varies depending on the kinds of the administration way, dosage form, administration site, excipient and additive, the pharmaceutical composition of the present invention includes from 0.01 to 100% by mass, in an embodiment, from 0.01 to 50% by mass, of one or more of the compound of the formula (I) or a salt thereof as an active ingredient.

The compound of the formula (I) or a salt thereof can be used in combination with various agents for treating or agents for preventing the above-described diseases for which the compound of the formula (I) or a salt thereof is considered to be effective. The combined preparation may be administered simultaneously, or separately and continuously or at a desired time interval. The preparations to be co-administered may be prepared separately, or may be a pharmaceutical composition containing various agents for treating or agents for preventing the above-described diseases for which the compound of the formula (I) or a salt thereof is considered to be effective and the compound of the formula (I) or a salt thereof.

## Examples

The production processes of the compound of the formula (I) or a salt thereof will be described below in more detail based on Examples. In this connection, the present invention is not limited to the compounds described in the following Examples. Furthermore, the production processes for the starting compounds will be described in Production Examples, and the production for the known compounds will be described in Reference Examples. Further, the production processes for the compound of the formula (I) or a salt thereof are not limited only to the production processes of the specific Examples as below, but the compound of the formula (I) or a salt thereof can be prepared by any combination of the production processes or the methods that are apparent to a person skilled in the art.

Production Example 1

7-fluoro-1H-indol-2,3-dione (1.651g) was dissolved into DMF (10 mL), sodium hydride (60% in oil, 600 mg) was added thereto under ice cooling, followed by stirring for 30 minutes, and 1-bromo-2-methylpropane (1.10 mL) was added thereto, followed by stirring for 4 hours. The solvent was evaporated under reduced pressure, then the residue was diluted with ethyl acetate, followed by washing with water and brine in this order. The organic layer was dried over anhydrous magnesium sulfate, the solvent was evaporated under reduced pressure, and the residue was purified by silica gel column chromatography (hexane:ethyl acetate=90:10) to obtain 7-fluoro-1-isobutyl-1H-indol-2,3-dione (1.032g).

Production Example 2

1,3-dihydro-2H-indol-2-one (600 mg) was dissolved into EtOH (15 mL), 1-methyl-1H-imidazole-2-carbaldehyde (794 mg) and piperidine (0.06 mL) was added thereto, followed by stirring at 75˚C for 1 hour. The precipitated solid was collected by filtration to obtain 3-[(1-methyl-1H-imidazol-2-yl)methylene]-1,3-dihydro-2H-indol-2-one (1.180 g).

Production Example 3

7-fluoro-1-isobutyl-1H-indol-2,3-dione (1.027g) was dissolved into THF (10 mL), ethyl(triphenylphosphoranylidene)acetate (1.779 g) was added thereto, followed by stirring at 60˚C for 10 hours. The solvent was evaporated under reduced pressure, and the residue was dissolved into ethyl acetate, followed by washing with saturated aqueous sodium bicarbonate and brine in this order. The organic layer was dried over anhydrous magnesium sulfate, and the solvent was evaporated under reduced pressure, and the residue was purified by silica gel column chromatography (hexane: ethyl acetate=90:10) to obtain ethyl (2E)-(7-fluoro-1-isobutyl-2-oxo-1,2-dihydro-3H-indol-3-ylidene) acetate (724 mg).

Production Example 4

Sodium hydride (50-72% in oil) was dissolved into EtOH (10 mL), ethyl (2E)-[1-(2-acetoxyethyl)-2-oxo-1,2-dihydro-3H-indol-3-ylidene]acetate (500 mg) was added thereto, followed by stirring at room temperature for 2 hours. 1M Hydrochloric acid was added to the reaction liquid, followed by extraction with ethyl acetate, and the organic layer was dried over anhydrous magnesium sulfate. The solvent was evaporated under reduced pressure, the residue was purified by silica gel column chromatography (hexane:ethyl acetate=10:1 to 1:1) to obtain yellow solid ethyl (2E)-[1-(2-hydroxyethyl)-2-oxo-1,2-dihydro-3H-indol-3-ylidene] acetate (52 mg).

In the same manner as Production Examples above, the compounds of Production Examples shown in Tables below were prepared, using corresponding starting materials, respectively. The structures of compounds of Production Examples are shown in Table 5 to Table 24, and the production processes and physical data thereof are shown in Table 44 to Table 46.

Example 1

Ethyl (2E)-(7-fluoro-1-isobutyl-2-oxo-1,2-dihydro-3H-indol-3-ylidene) acetate (626 mg) was suspended in EtOH (7 mL), 1M aqueous sodium hydroxide solution (2.2 mL) was added thereto, followed by stirring at room temperature for 10 minutes. Under ice cooling, 1M hydrochloric acid (2.2 mL) was added to the reaction liquid, followed by stirring at room temperature for 20 minutes, and the solvent was evaporated under reduced pressure. The residue was washed with water, and purified by silica gel column chromatography (chloroform:MeOH:formic acid=97:3:0.3) to obtain yellow solid (2E)-(7-fluoro-1-isobutyl-2-oxo-1,2-dihydro-3H-indol-3-ylidene)acetic acid (150 mg).

Example 2

(2E)-(1-benzyl-2-oxo-1,2-dihydro-3H-indol-3-ylidene)acetic acid (300 mg) was suspended in DMF (10 mL), 1,1'-carbonyl bis-1H-imidazole (209 mg) was added thereto, followed by stirring at room temperature for 2 hours. Then, methanesulfonamide (307 mg) and 1,8-diazabicyclo[5.4.0]undec-7-ene (0.48 mL) was added thereto, followed by stirring at room temperature for 2 hours. Water was added to the reaction liquid, followed by extraction with ethyl acetate, and the organic layer was dried over anhydrous magnesium sulfate. The solvent was evaporated under reduced pressure, and the residue was purified by silica gel column chromatography (Chloroform:MeOH=10:1) to obtain yellow solid (2E)-2-(1-benzyl-2-oxo-1,2-dihydro-3H-indol-3-ylidene)-N-(methylsulfonyl)acetamide (40 mg).

Example 3

Methyl 3-{[(3E)-3-(2-tert-butoxy-2-oxoethylidene)-2-oxo-2,3-dihydro-1H-indol-1-yl]methyl}benzoate (2.00 g) was dissolved into dichloroethane (40 mL), and trifluoroacetic acid (40 mL) was added thereto, followed by stirring at room temperature for 1 hour. The solvent was evaporated under reduced pressure, and the residue was diluted with ethyl acetate, followed by washing with water. The organic layer was dried over anhydrous magnesium sulfate, and the solvent was evaporated under reduced pressure to obtain orange solid (2E)-(1-[3-(methoxycarbonyl)benzyl]-2-oxo-1,2-dihydro-3H-indol-3-ylidene}acetic acid (1.70 g).

Example 4

(2E)- {1-[3-(methoxycarbonyl)benzyl]-2-oxo-1,2-dihydro-3H-indol-3-ylidene}acetic acid (960 mg) was dissolved into dichloromethane (20 mL), and oxalyl chloride (0.30 mL) was added thereto, followed by stirring at room temperature for 1 hour. The solvent was evaporated under reduced pressure and the residue was dissolved into dichloromethane (20 mL), 28% ammonia water (5 mL) was added thereto, and the precipitated solid was collected by filtration to obtain yellow solid methyl 3-{[(3E}-3-(2-amino-2-oxoethylidene)-2-oxo-2,3-dihydro-1H-indol-1-yl]methyl)benzoate (220 mg).

Example 5

(2E)-(1-benzyl-2-oxo-1,2-dihydro-3H-indol-3-ylidene)acetic acid (390 mg) was suspended in DMF (10 mL), and HOBt (283 mg) was added thereto, followed by stirring at room temperature for 1 hour, then EDCI·HCl (325 mg) and 2-(aminooxy)tetrahydro-2H-pyrane (196 mg) were added thereto, followed by stirring at room temperature for 2 hours. Water was added to the reaction liquid, followed by extraction with chloroform, and the organic layer was dried over anhydrous magnesium sulfate. The solvent was evaporated under reduced pressure, and the residue was purified by silica gel column chromatography (chloroform:MeOH=10:1) to obtain a yellow solid product. The product was dissolved into 1M hydrochloric acid, followed by stirring at room temperature for 3 hours, and the precipitated solid was collected by filtration to obtain pale yellow solid (2E)-2-(1-benzyl-2-oxo-1,2-dihydro-3H-indol-3-ylidene)-N-hydroxyacetamide (22 mg).

Example 6

3-[(1-methyl-1H-imidazol-2-yl)methylene]-1,3-dihydro-2H-indol-2-one (300 mg) was dissolved into DMF (10 mL), potassium carbonate (202 mg) and (bromomethyl)benzene (0.17 mL) were added thereto, followed by stirring at room temperature overnight, and potassium carbonate (202 mg) and (bromomethyl)benzene (0.17 mL) were added thereto, followed by stirring at room temperature overnight. Water was added to the reaction liquid, and the precipitated solid was collected by filtration to obtain yellow solid 1-benzyl-3-[(1-methyl-1H-imidazol-2-yl)methylene]-1,3-dihydro-2H-indol-2-one (405 mg).

Example 7

Methyl 3-{[(3E)-3-(2-amino-2-oxoethylidene)-2-oxo-2,3-dihydro-1H-indol-1-yl]methyl}benzoate (100 mg) was suspended in 35% hydrochloric acid (5 mL), followed by stirring at 70˚C for 5 hours. The reaction liquid was left to cool to room temperature, then the precipitated solid was collected by filtration, followed by washing with EtOH to obtain yellow solid 3-{[(3E)-3-(2-amino-2-oxoethylidene)-2-oxo-2,3-dihydro-1H-indol-1-yl]methyl}benzoate (67 mg).

In the same manner as Examples above, the compounds of Examples shown in Tables below were prepared, using corresponding starting materials, respectively. The structures of Example compounds are shown in Table 25 to Table 43, and production processes and physical data thereof are shown in Table 47 to Table 52.

Further, the following abbreviations are used in the following tables. Pr: Production Example number (Production Example where "/Cl" is described after Production Example number, represents that the Production Example compound was isolated as hydrochloride.), Ex: Example number (Example where "/Cl" is described after Example number, represents that the Example compound was isolated as hydrochloride.), Structure: structural formula, Syn: production process (among the Examples or Production Examples above, Production Example number or Example number produced in the same manner is shown. For example, it shows that the compound of Production Example 5 was produced in the same manner as the compound of Production Example 1). Data: physicochemical data (NMR-D: δ(ppm) in 1H NMR among DMSO-$d_6$, representing values measured in FAB+:FAB-MS (cation), ESI+:ESI-MS (cation), EI:EI-MS (cation), APCI+:APCI-MS (cation), APCI/ESI+:APCI-MS (cation) or ESI-MS (cation), representing values measured in APCI/ESI-:APCI-MS (anion) or ESI-MS (anion), m.p.: melting point). In this connection, the dotted line (Production Example

2, Production Example 45, Example 6 and Example 21) in a structural formula represents a double bond, and whether it is E-form or Z-form is not determined, but it shows that it is an isomer of either thereof. Furthermore, the cross line (Example 16) in a structural formula represents a double bond, and it shows that it is a mixture of E-form and Z-form.

[Table 5]

| Pr | Structure |
|---|---|
| 1 | 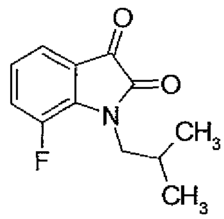 |
| 2 | 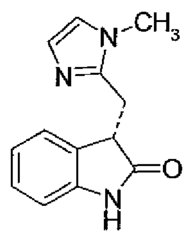 |
| 3 | 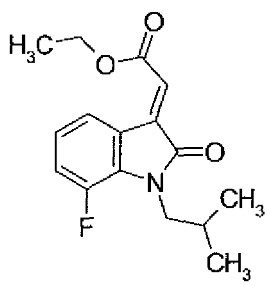 |
| 4 | 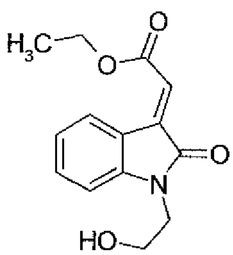 |
| 5 | 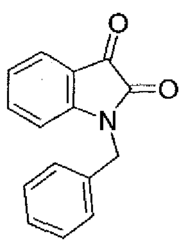 |

[Table 6]

| Pr | Structure |
|---|---|
| 6 | 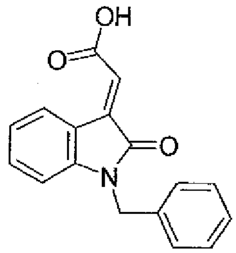 |
| 7 | 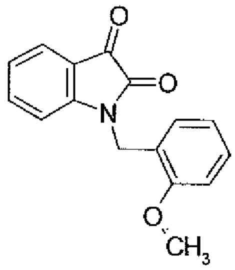 |
| 8 | 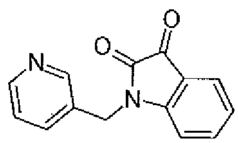 |
| 9 | 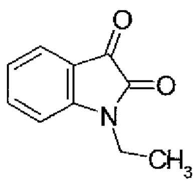 |
| 10 | 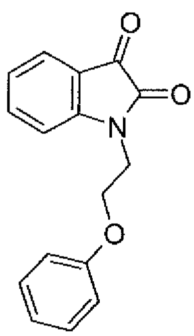 |

[Table 7]

| Pr | Structure |
|---|---|
| 11 | 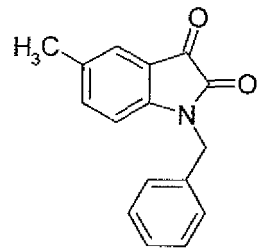 |
| 12 | 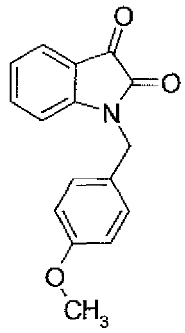 |
| 13 | 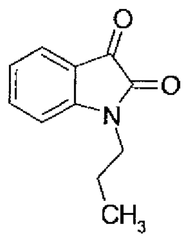 |
| 14 | 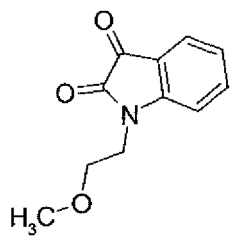 |
| 15 | 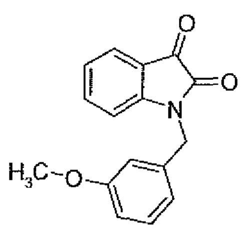 |

[Table 8]

| Pr | Structure |
|---|---|
| 16 | 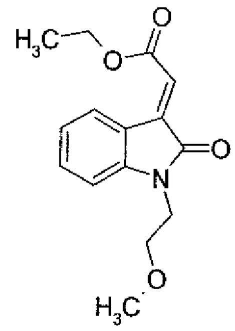 |
| 17 | 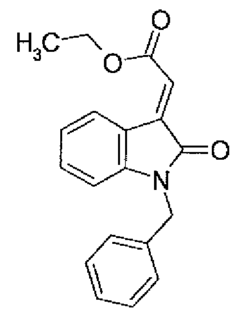 |
| 18 | 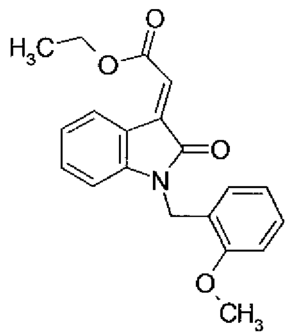 |
| 19 | 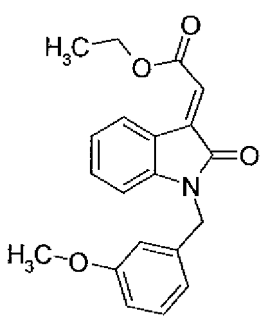 |

[Table 9]

| Pr | Structure |
|---|---|
| 20 | 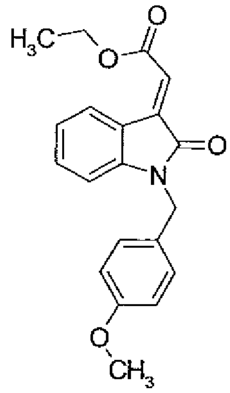 |
| 21 | 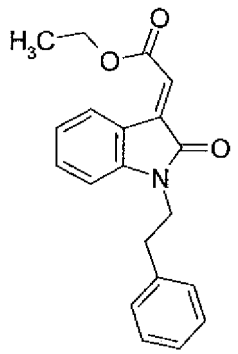 |
| 22 | 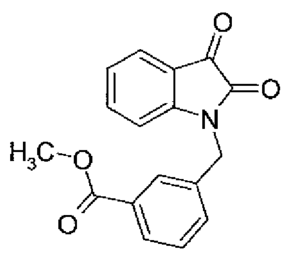 |
| 23 | 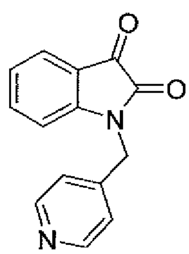 |

[Table 10]

| Pr | Structure |
|---|---|
| 24 | 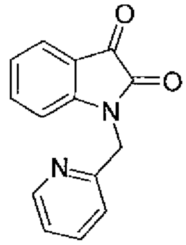 |
| 25 | 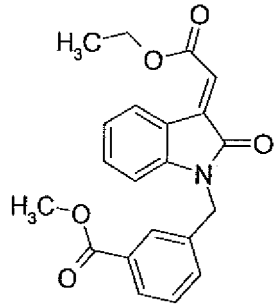 |
| 26/Cl | 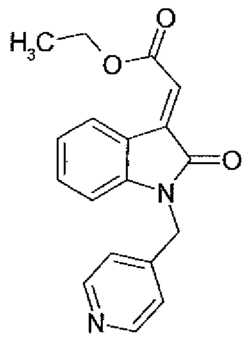 |
| 27/Cl | 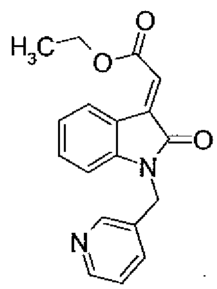 |

[Table 11]

| Pr | Structure |
|---|---|
| 28 | 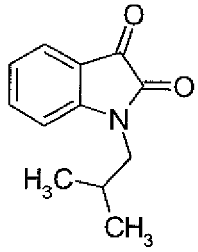 |
| 29 | 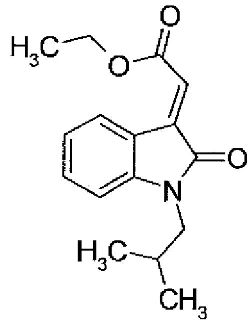 |
| 30 | 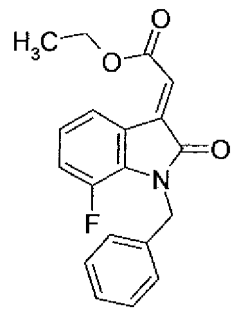 |
| 31 | 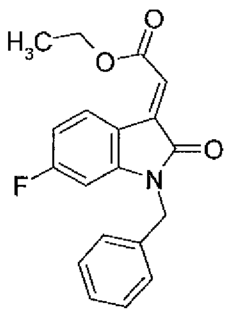 |

[Table 12]

| Pr | Structure |
|---|---|
| 32 | 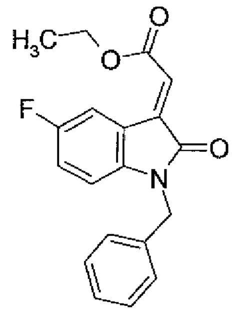 |
| 33 | 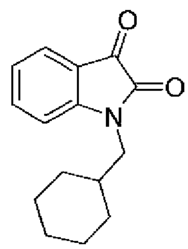 |
| 34 | 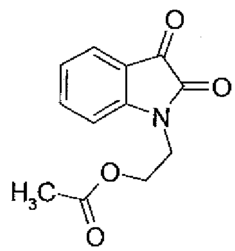 |
| 35/Cl | 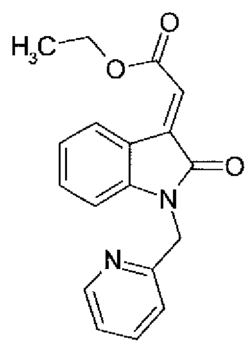 |

[Table 13]

| Pr | Structure |
|---|---|
| 36 | 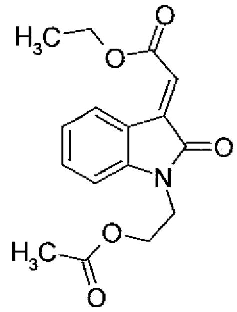 |
| 37 | 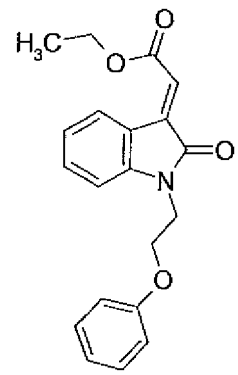 |
| 38 | 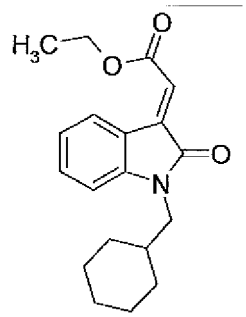 |
| 39 | 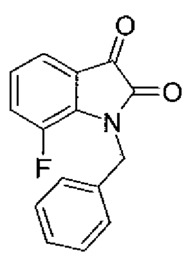 |

Table 14]

| Pr | Structure |
|---|---|
| 40 | 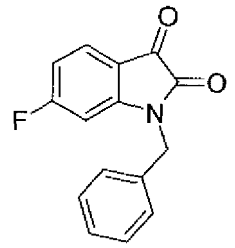 |
| 41 | 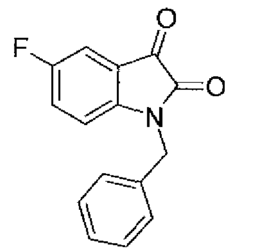 |
| 42 | 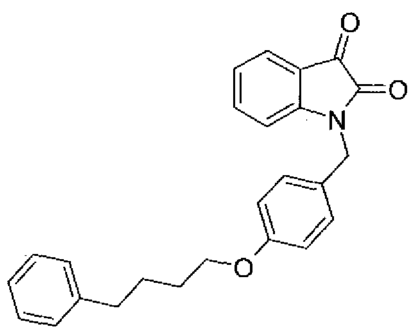 |
| 43 | 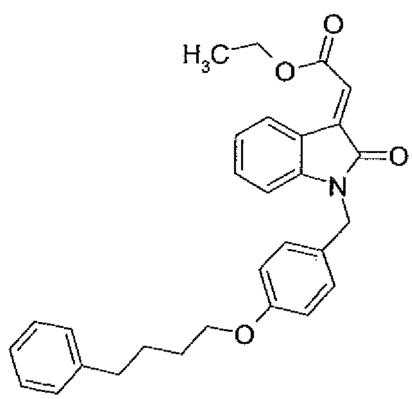 |

[Table 15]

| Pr | Structure |
|---|---|
| 44 | 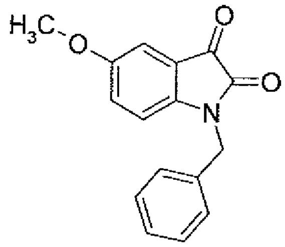 |
| 45 | 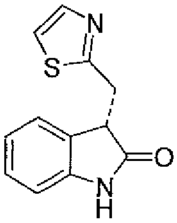 |
| 46 | 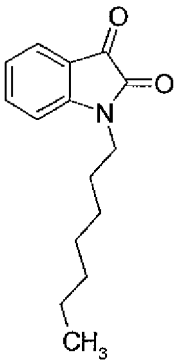 |
| 47 | 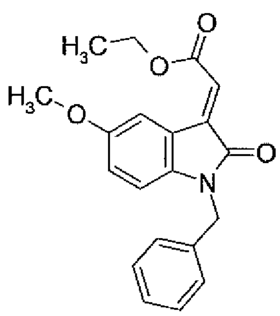 |

[Table 16]

| Pr | Structure |
|---|---|
| 48 | 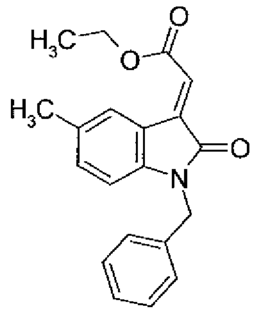 |
| 49 | 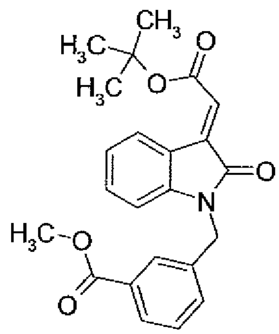 |
| 50 | 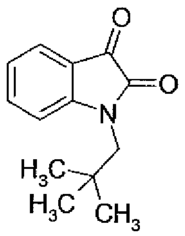 |
| 51 | 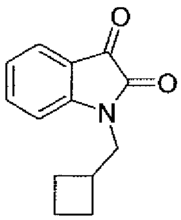 |

[Table 17]

| Pr | Structure |
|---|---|
| 52 | 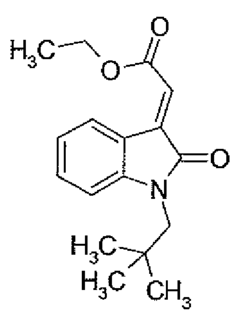 |

(continued)

| Pr | Structure |
|---|---|
| 53 | 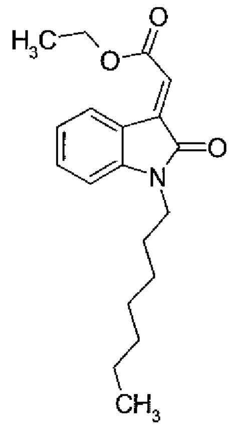 |
| 54 | 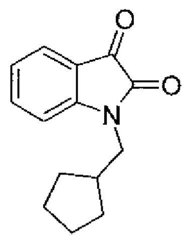 |
| 55 | 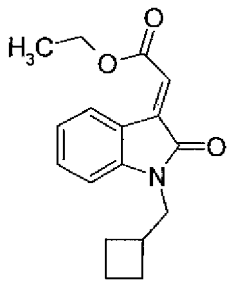 |

[Table 18]

| Pr | Structure |
|---|---|
| 56 | 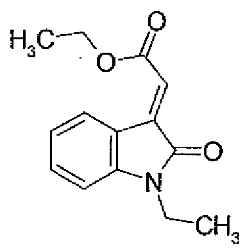 |

(continued)

| Pr | Structure |
|---|---|
| 57 | 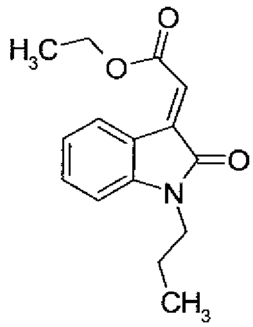 |
| 58 | 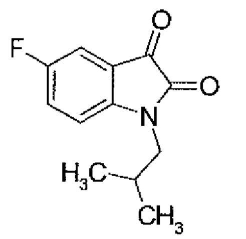 |
| 59 | 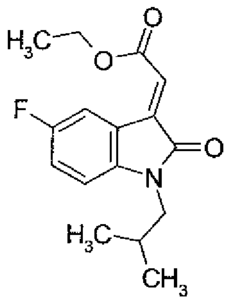 |

[Table 19]

| Pr | Structure |
|---|---|
| 60 | 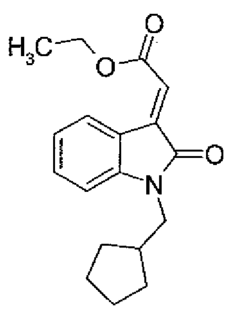 |
| 61 | 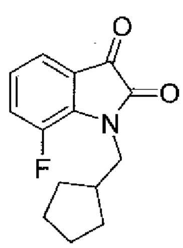 |

(continued)

| Pr | Structure |
|---|---|
| 62 | 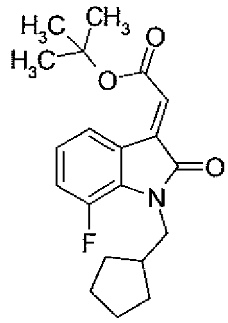 |
| 63 | 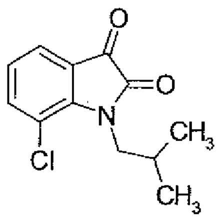 |

[Table 20]

| Pr | Structure |
|---|---|
| 64 | 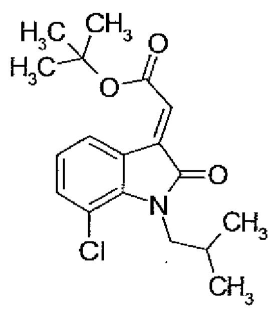 |
| 65 | 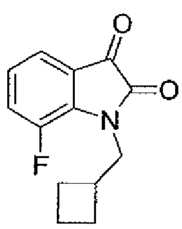 |
| 66 | 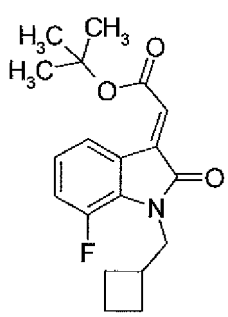 |

(continued)

| Pr | Structure |
|---|---|
| 67 | 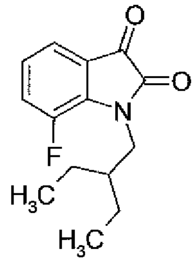 |
| 68 | 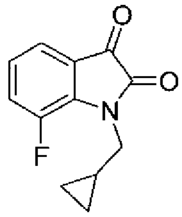 |

[Table 21]

| Pr | Structure |
|---|---|
| 69 | 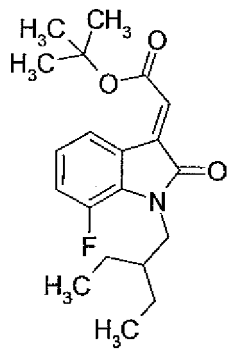 |
| 70 | 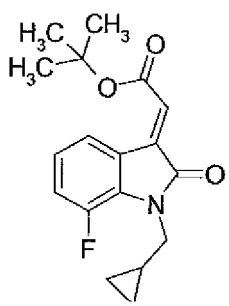 |
| 71 | 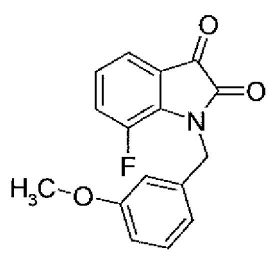 |

(continued)

| Pr | Structure |
|---|---|
| 72 | 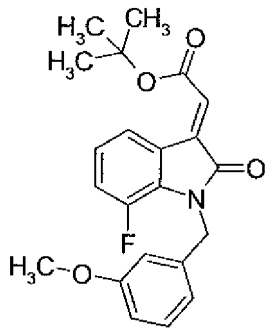 |

[Table 22]

| Pr | Structure |
|---|---|
| 73 | 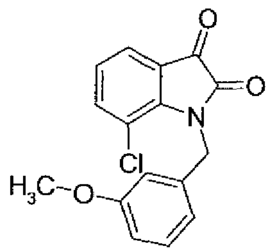 |
| 74 | 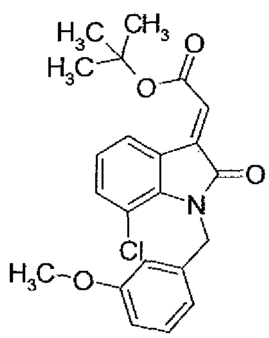 |
| 75 | 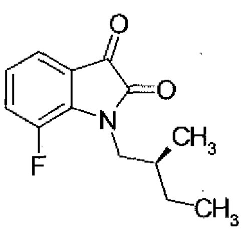 |

(continued)

| Pr | Structure |
|---|---|
| 76 | 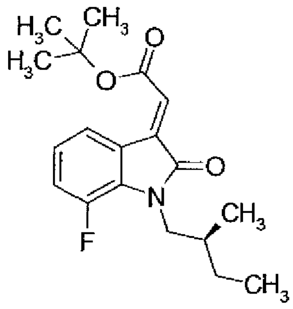 |

[Table 23]

| Pr | Structure |
|---|---|
| 77 | 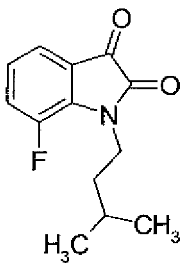 |
| 78 | 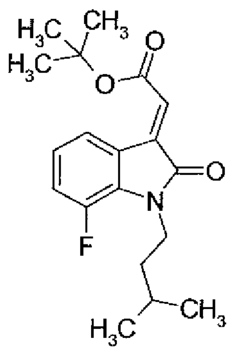 |
| 79 | 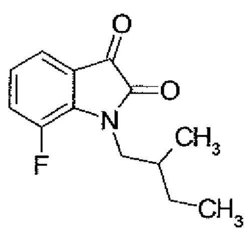 |

(continued)

| Pr | Structure |
|---|---|
| 80 | 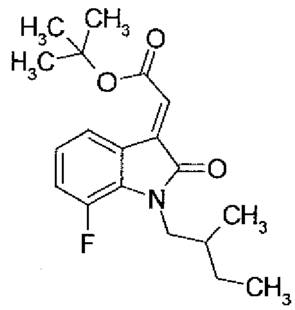 |

[Table 24]

| Pr | Structure |
|---|---|
| 81 | 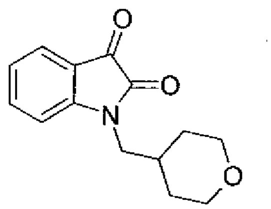 |
| 82 | 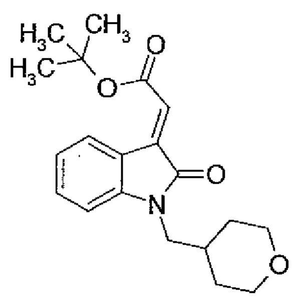 |
| 83 | 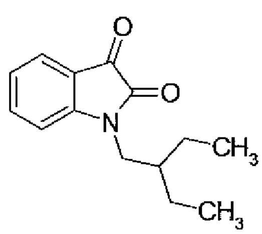 |

(continued)

| Pr | Structure |
|---|---|
| 84 | 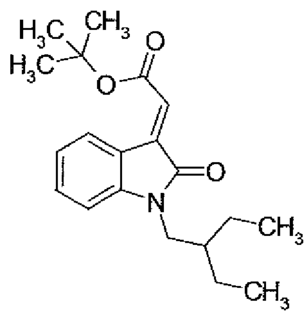 |

[Table 25]

| Ex | Structure |
|---|---|
| 1 | 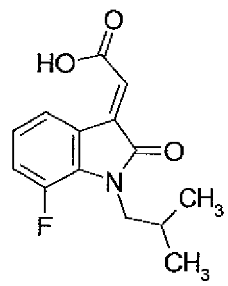 |
| 2 | 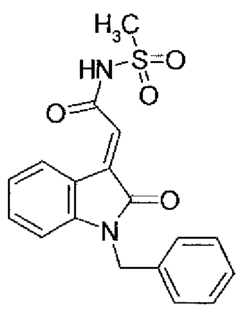 |
| 3 | 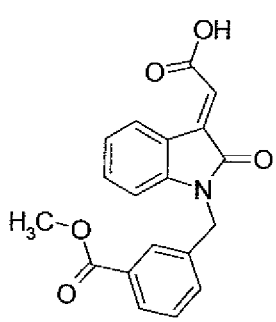 |

(continued)

| Ex | Structure |
|---|---|
| 4 | 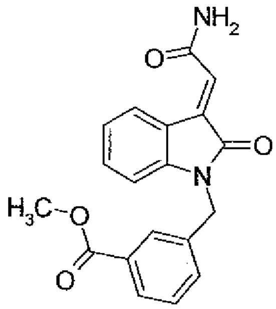 |

[Table 26]

| Ex | Structure |
|---|---|
| 5 | 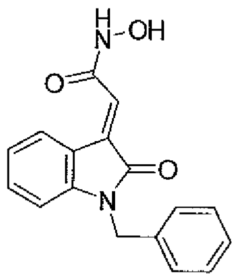 |
| 6 | 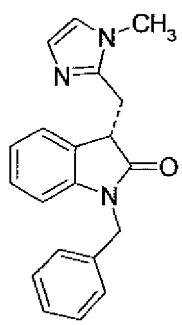 |
| 7 | 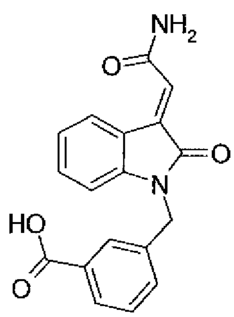 |

(continued)

| Ex | Structure |
|---|---|
| 8 | 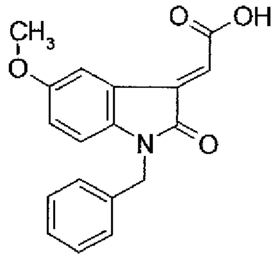 |

[Table 27]

| Ex | Structure |
|---|---|
| 9 | 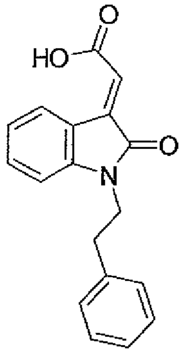 |
| 10 | 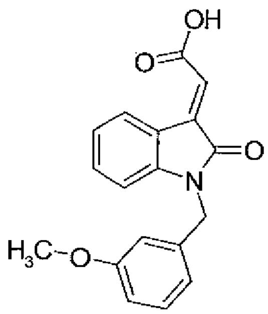 |
| 11 | 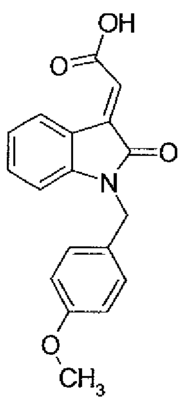 |

(continued)

| Ex | Structure |
|---|---|
| 12 | 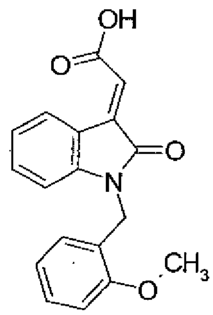 |

[Table 28]

| Ex | Structure |
|---|---|
| 13 | 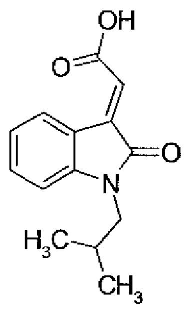 |
| 14 | 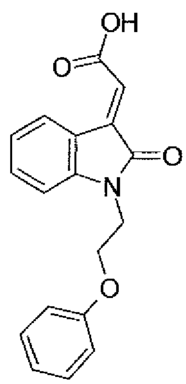 |
| 15 | 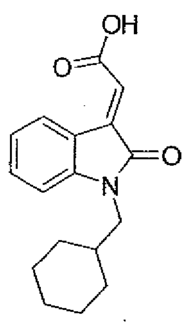 |

(continued)

| Ex | Structure |
|---|---|
| 16 | 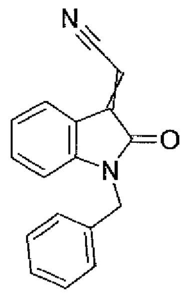 (E-, Z-mixture) |

[Table 29]

| Ex | Structure |
|---|---|
| 17 | 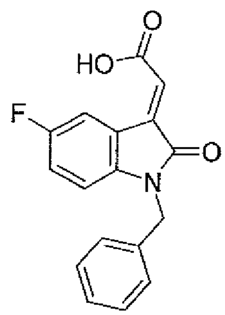 |
| 18 | 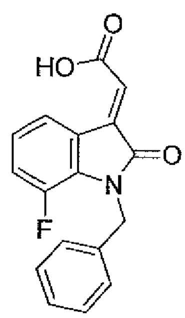 |
| 19 | 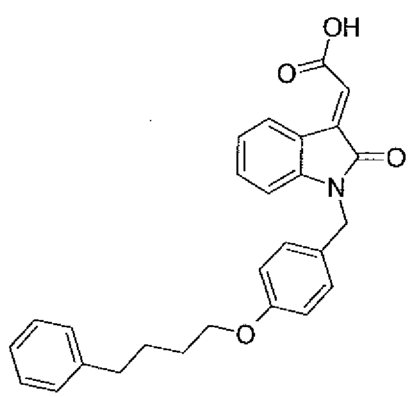 |

(continued)

| Ex | Structure |
|---|---|
| 20 | 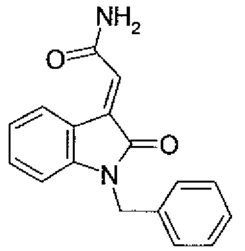 |

[Table 30]

| Ex | Structure |
|---|---|
| 21 | 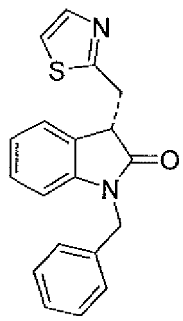 |
| 22 | 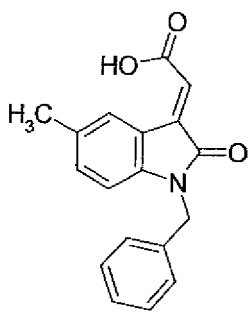 |
| 23 | 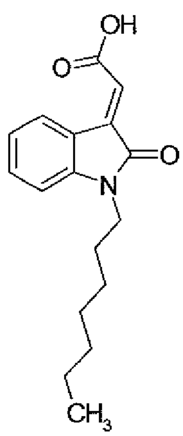 |

(continued)

| Ex | Structure |
|---|---|
| 24 | 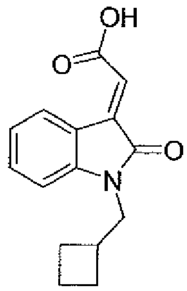 |

[Table 31]

| Ex | Structure |
|---|---|
| 25 | 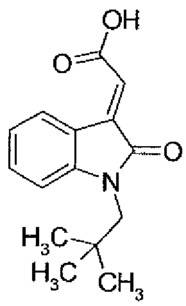 |
| 26 | 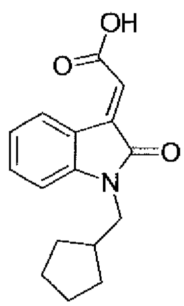 |
| 27 | 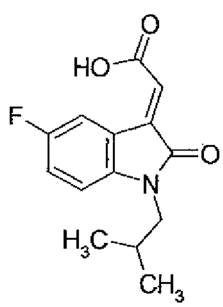 |

(continued)

| Ex | Structure |
|---|---|
| 28 | 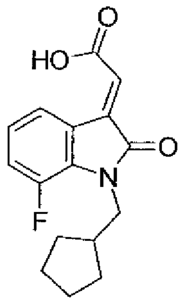 |

[Table 32]

| Ex | Structure |
|---|---|
| 29 | 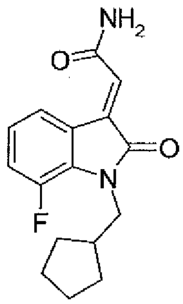 |
| 30 | 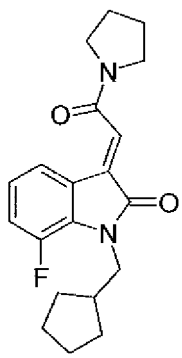 |
| 31 | 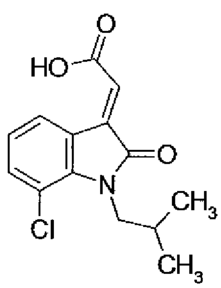 |

(continued)

| Ex | Structure |
|---|---|
| 32 | 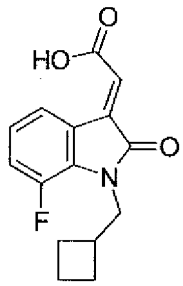 |

[Table 33]

| Ex | Structure |
|---|---|
| 33 | 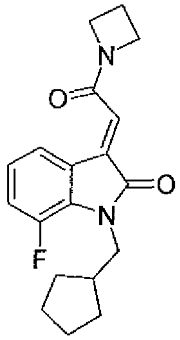 |
| 34 | 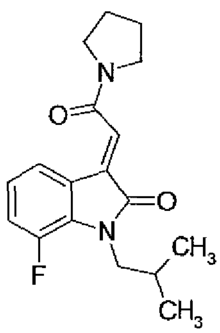 |
| 35 | 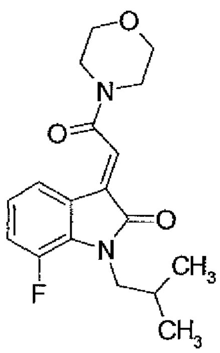 |

(continued)

| Ex | Structure |
|---|---|
| 36 | 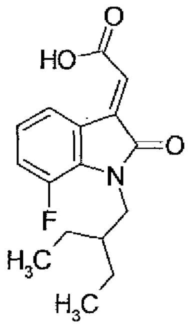 |

[Table 34]

| Ex | Structure |
|---|---|
| 37 | 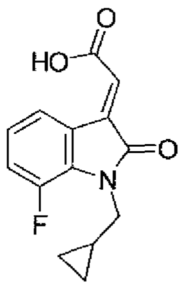 |
| 38 | 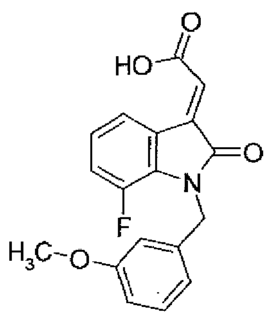 |
| 39 | 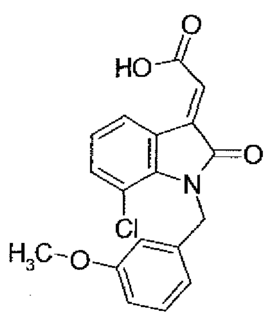 |

(continued)

| Ex | Structure |
|---|---|
| 40 | 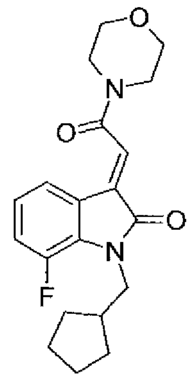 |

[Table 35]

| Ex | Structure |
|---|---|
| 41 | 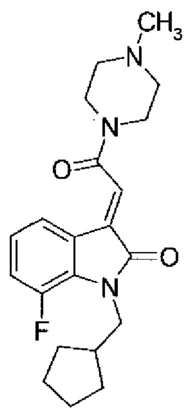 |
| 42 | 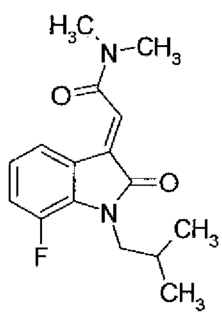 |
| 43 | 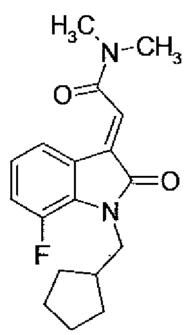 |

[Table 36]

| Ex | Structure |
|---|---|
| 44 | 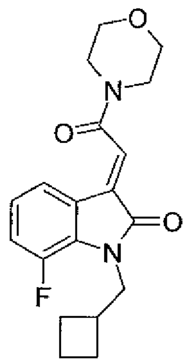 |
| 45 | 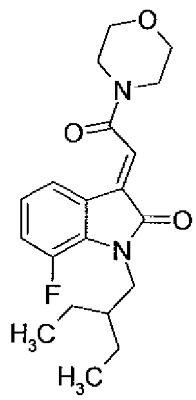 |
| 46 | 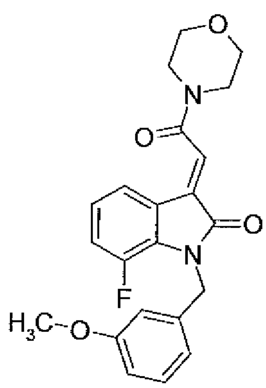 |

[Table 37]

| Ex | Structure |
|---|---|
| 47 | 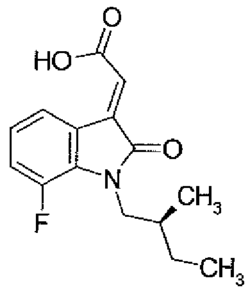 |
| 48 | 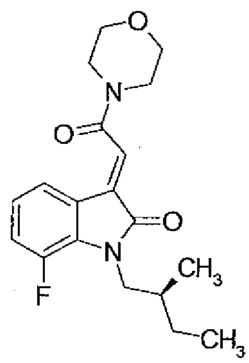 |
| 49 | 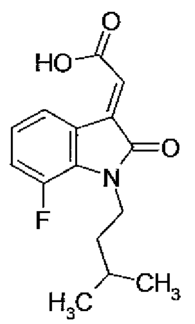 |
| 50 | 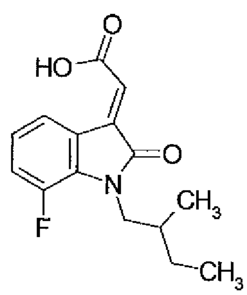 |

[Table 38]

| Ex | Structure |
|---|---|
| 51 | 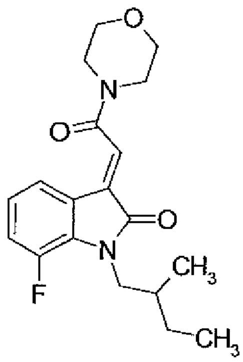 |
| 52 | 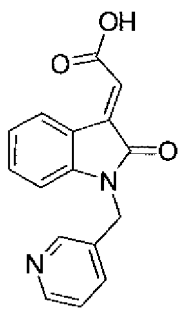 |
| 53 | 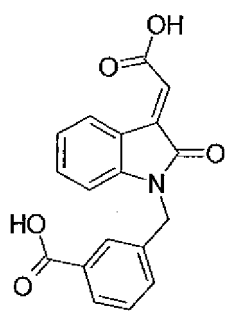 |

[Table 39]

| Ex | Structure |
|---|---|
| 54/Cl | 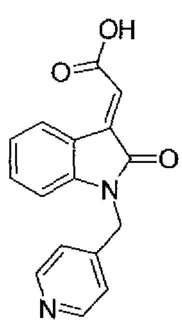 |

(continued)

| Ex | Structure |
|---|---|
| 55 | 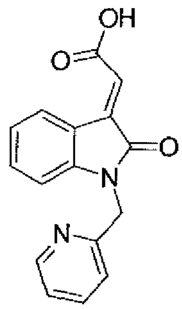 |
| 56 | 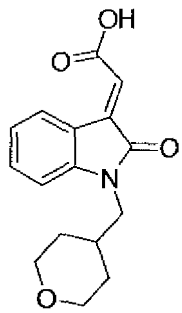 |

[Table 40]

| Ex | Structure |
|---|---|
| 57 | 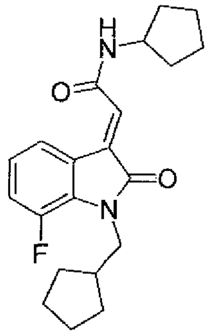 |
| 58 | 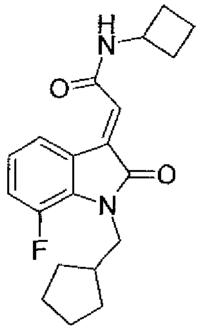 |

(continued)

| Ex | Structure |
|---|---|
| 59 | 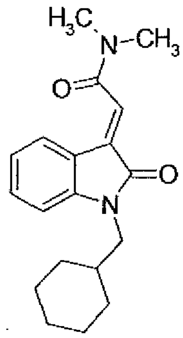 |

[Table 41]

| Ex | Structure |
|---|---|
| 60 | 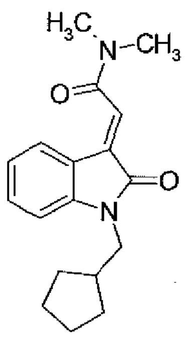 |
| 61 | 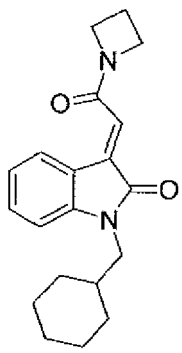 |

(continued)

| Ex | Structure |
|---|---|
| 62 | 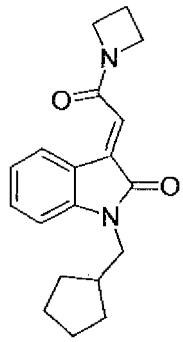 |

[Table 42]

| Ex | Structure |
|---|---|
| 63 | 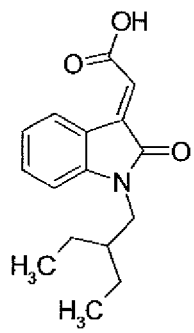 |
| 64 | 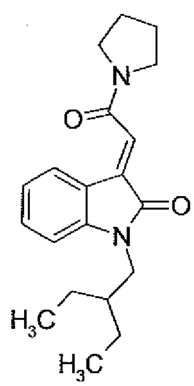 |

(continued)

| Ex | Structure |
|---|---|
| 65 | 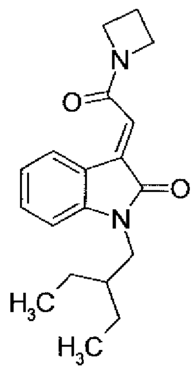 |

[Table 43]

| Ex | Structure |
|---|---|
| 66 | 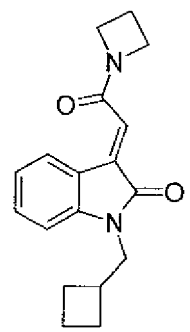 |
| 67 | 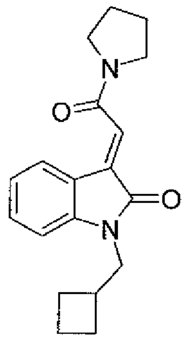 |

[Table 44]

| Pr | Syn | Data |
|---|---|---|
| 1 | Pr.1 | ESI+:222. |
| 2 | Pr.2 | ESI+:226. |
| 3 | Pr.3 | FAB+:292. |
| 4 | Pr.4 | EI:261. |

(continued)

| Pr | Syn | Data |
|---|---|---|
| 5 | Pr.1 | ESI+:238. |
| 6 | Ex.1 | ESI+:280. |
| 7 | Pr.1 | ESI+:268. |
| 8 | Pr.1 | ESI+:239. |
| 9 | Pr.1 | ESI+:176. |
| 10 | Pr.1 | ESI+:268. |
| 11 | Pr.1 | ESI+:252. |
| 12 | Pr.1 | ESI+:267[M]. |
| 13 | Pr.1 | ESI+:190. |
| 14 | Pr.1 | ESI+:206. |
| 15 | Pr.1 | ESI+:268. |
| 16 | Pr.3 | ESI+:276. |
| 17 | Pr.3 | ESI+:308. |
| 18 | Pr.3 | ESI+:337[M]. |
| 19 | Pr.3 | ESI+:338. |
| 20 | Pr.3 | ESI+:338. |
| 21 | Pr.3 | ESI+:322. |
| 22 | Pr.1 | ESI+:296. |
| 23 | Pr.1 | ESI+:239. |
| 24 | Pr.1 | ESI+:239. |
| 25 | Pr.3 | EI:365. |
| 26 | Pr.3 | ESI+:309. |
| 27 | Pr.3 | ESI+:309. |
| 28 | Pr.1 | APCI+:203[M]. |
| 29 | Pr.3 | ESI+:274. |
| 30 | Pr.3 | EI:325. |

[Table 45]

| Pr | Syn | Data |
|---|---|---|
| 31 | Pr.3 | EI:325. |
| 32 | Pr.3 | EI:325. |
| 33 | Pr.1 | ESI+:244. |
| 34 | Pr.1 | ESI+:234. |
| 35 | Pr.3 | ESI+:309. |
| 36 | Pr.3 | ESI+:304. |
| 37 | Pr.3 | ESI+:338. |
| 38 | Pr.3 | ESI+:314. |

(continued)

| Pr | Syn | Data |
|---|---|---|
| 39 | Pr.1 | ESI+:255[M]. |
| 40 | Pr.1 | ESI+:255[M], |
| 41 | Pr.1 | ESI+:255[M]. |
| 42 | Pr.1 | ESI+:386. |
| 43 | Pr.3 | ESI+:456. |
| 44 | Pr.1 | ESI+:268. |
| 45 | Pr.2 | ESI+:229. |
| 46 | Pr.1 | ESI+:246. |
| 47 | Pr.3 | ESI+:338. |
| 48 | Pr.3 | ESI+:322. |
| 49 | Pr.3 | NMR-D:1.54(9H,s),3.84(3H,s),5.04(2H,s),6.71(1H,s),7. 00(1H,d,J=7.89Hz),7.05-7.12(1H,m), 7.33-7.41(1H,m), 7.49(1H,t,J=7.74Hz),7.60(1H,d,J=7.74Hz),7.87(1H,d,J =7.774Hz),7.93(1H,s),8.35 (1H,d,J=7.68Hz). |
| 50 | Pr.1 | ESI+:218. |
| 51 | Pr.1 | ESI+:216. |
| 52 | Pr.3 | ESI+:288. |
| 53 | Pr.3 | ESI+:316. |
| 54 | Pr.1 | ESI+:230. |
| 55 | Pr.3 | ESI+:286. |
| 56 | Pr.3 | ESI+:246. |
| 57 | Pr.3 | FAB+:260. |
| 58 | Pr.1 | ESI+:222. |

[Table 46]

| Pr | Syn | Data |
|---|---|---|
| 59 | Pr. 3 | FAB+:292. |
| 60 | Pr.3 | ESI+:300. |
| 61 | Pr.1 | ESI+:248. |
| 62 | Pr.3 | APCI+:346. |
| 63 | Pr.1 | ESI+:238. |
| 64 | Pr.3 | ESI+:336. |
| 65 | Pr.1 | ESI+:234. |
| 66 | Pr.3 | ESI+:332. |
| 67 | Pr.1 | APCI/ESI+:249[M]. |
| 68 | Pr.1 | APCI/ESI+:219[M]. |
| 69 | Pr.3 | APCI/ESI+:348. |
| 70 | Pr.3 | APCI/ESI+:318. |

(continued)

| Pr | Syn | Data |
|---|---|---|
| 71 | Pr.1 | APCI/ESI-:285[M]. |
| 72 | Pr.3 | APCI/ESI+:384. |
| 73 | Pr.1 | APCI/ESI-:301[M]. |
| 74 | Pr.3 | APCI/ESI+:400. |
| 75 | Pr.1 | APCI/ESI+:236. |
| 76 | Pr.3 | APCI/ESI+:334. |
| 77 | Pr.1 | APCI/ESI+:236. |
| 78 | Pr.3 | APCI/ESI+:334. |
| 79 | Pr.1 | APCI/ESI+:236. |
| 80 | Pr.3 | APCI/ESI+:334. |
| 81 | Pr.1 | ESI+:246. |
| 82 | Pr.3 | ESI+:344. |
| 83 | Pr.1 | ESI+:232. |
| 84 | Pr.3 | APCI/ESI+:330. |

[Table 47]

| Ex | Syn | Data |
|---|---|---|
| 1 | Ex.1 | FAB+:264. |
| 2 | Ex.2 | FAB+:357. |
| 3 | Ex.3 | FAB+:338. |
| 4 | Ex.4 | FAB+:337. |
| 5 | Ex.5 | FAB+:295. |
| 6 | Ex.6 | ESI+:316. |
| 7 | Ex.7 | EI:323. |
| 8 | Ex.1 | ESI+:310. |
| 9 | Ex.1 | FAB+:294. |
| 10 | Ex.1 | ESI+:310. |
| 11 | Ex.1 | ESI+:310. |
| 12 | Ex.1 | FAB+:310. |
| 13 | Ex.1 | FAB+:246. |
| 14 | Ex.1 | FAB+:310. |
| 15 | Ex.1 | ESI+:286. |
| 16 | Pr.3 | FAB+:261. |
| 17 | Ex.1 | FAB+:298. |
| 18 | Ex.1 | FAB+:298. |
| 19 | Ex.1 | FAB+:428. |
| 20 | Ex.4 | ESI+:279. |

(continued)

| Ex | Syn | Data |
|---|---|---|
| 21 | Ex.6 | ESI+:319. |
| 22 | Ex.1 | ESI+:294. |
| 23 | Ex.1 | FAB+:288. |
| 24 | Ex.1 | ESI+:258. |
| 25 | Ex.1 | FAB+:260. |
| 26 | Ex.1 | FAB+:272. |
| 27 | Ex.1 | FAB+:264. |
| 28 | Ex.3 | ESI+:290. |
| 29 | Ex.4 | ESI+:289. |
| 30 | Ex.4 | ESI+:343. |

[Table 48]

| Ex | Syn | Data |
|---|---|---|
| 31 | Ex.3 | ESI+:280. |
| 32 | Ex.3 | ESI+:276. |
| 33 | Ex.4 | ESI+:329. |
| 34 | Ex.4 | ESI+:317. |
| 35 | Ex.4 | ESI+:333. |
| 36 | Ex.3 | ESI+:292. |
| 37 | Ex.3 | ESI+:262. |
| 38 | Ex.3 | ESI+:328. |
| 39 | Ex.3 | ESI+:343[M]. |
| 40 | Ex.4 | ESI+:359. |
| 41 | Ex.4 | ESI+:372. |
| 42 | Ex.4 | ESI+:291. |
| 43 | Ex.4 | ESI+:317. |
| 44 | Ex.4 | ESI+:345. |
| 45 | Ex.4 | ESI+:361. |
| 46 | Ex.4 | ESI+:397. |
| 47 | Ex.3 | ESI+:278. |
| 48 | Ex.4 | ESI+:347. |
| 49 | Ex.3 | ESI+:278. |
| 50 | Ex.3 | ESI+:278. |
| 51 | Ex.4 | ESI+:347. |
| 52 | Ex.1 | ESI+:281. |
| 53 | Ex.1 | ESI+:323. |
| 54 | Ex.1 | ESI+:281. |

(continued)

| Ex | Syn | Data |
|---|---|---|
| 55 | Ex.1 | ESI+:281. |
| 56 | Ex.3 | ESI+:288. |
| 57 | Ex.4 | ESI+:357. |
| 58 | Ex.4 | ESI+:343. |
| 59 | Ex.4 | ESI+:313. |
| 60 | Ex.4 | ESI+:299. |

[Table 49]

| Ex | Syn | Data |
|---|---|---|
| 61 | Ex.4 | ESI+:325. |
| 62 | Ex.4 | ESI+:311. |
| 63 | Ex.3 | ESI+:274. |
| 64 | Ex.4 | ESI+:327. |
| 65 | Ex.4 | ESI+:313. |
| 66 | Ex.4 | ESI+:297. |
| 67 | Ex.4 | ESI+:311. |

[Table 50]

| Ex | Data |
|---|---|
| 1 | NMR-D:0.89(6H,d,J=6.7Hz),1.95,2.02(1H,m),3.60-3.63(2H,m),6.7 9(1H,s),7.07-7.12(1H,m),7.32-7.37(1H, m),8.21(1H,d,J=7.8Hz),13. 57(1H,br-s).<br>m.p.:153-155˚C |
| 4 | NMR-D:3.83(3H,s),5.04(2H,s),6.96(1H,d,J=5.85Hz),7.01-7.06(2H, m),7.28-7.34(1H,m),7.47-7.53(1H,m), 7.60(1H,d,J=5.97Hz),7.66(1 H,s),7.86(1H,d,J=5.85Hz),7.92(1H,s),8.19(1H,s),8.55(1H,d,J=5.22 Hz). |
| 13 | NMR-D:0.88(6H,d,J=4.92Hz),2.00-2.10(1H,m),3.52(2H,d,J=5.52H z),6.70(1H,s),7.04-7.13(2H,m),7.37-7.45 (1H,m),5.35(1H,d,J=5.37 Hz),13.39(1H,br-s). |
| 15 | NMR-D:0.90-1.2(6H,m),1.50-1.80(6H,m),3.54(2H,d,J=4.00Hz),5. 27(1H,brs),6.69(1H,s),7.04-7.13(2H,m), 7.46(1H,t,J=8.00Hz),8.34 (1H,d,J=8.00Hz).<br>m.p.:137-139˚C |
| 17 | NMR-D:4.95(2H,s),6.83(1H,s),6.96-7.00(1H,m),7.23-7,36(6H,m), 8.18-8.21(1H,m),13.61(1H,brs).<br>m.p.:177-179˚C |
| 18 | NMR-D:5.04(2H,s),6.87(1H,s),7.07-7.12(1H,m),7.24-7.35(6H,m), 8.21-8.23(1H,m),13.61(1H,brs).<br>m.p.:168-170˚C |
| 24 | NMR-D:1.68-1.87(4H,m),1.88-2.01(2H,m),2.62-2.76(1H,m),3.75(2 H,d,J=5.34Hz),6.96(1H,s),7.03-7.12(2H, m),7.37-7.44(1H,m),8.33 (1H,d,J=5.37Hz),13.40(1H,br-s). |
| 25 | NMR-D:0.91(9H,s),3.53(2H,s),6.69(1H,s),7.02-7.09(1H,m),7.16(1 H,d,7=5.94Hz),7.37-7.43(1H,m),8.34(1H, d,J=5.76Hz),13.39(1H,b r-s). |

(continued)

| Ex | Data |
|---|---|
| 26 | NMR-D:1.19-1.32(2H,m),1.40-1.55(2H,m),1.54-1.70(4H,m),2.24-2.37(1H,m),3.63(2H,d,J=5.73Hz),6.69(1H, s),7.04-7.09(1H,m),7.1 2(1H,d,J=5.91Hz),7.40-7.44(1H,m),8.34(1H,d,J=5.28Hz),13.40(1 H,br-s). m.p.:140-142˚C |
| 27 | NMR-D:0.88(6H,d,J=6.7Hz),1.99-2.07(1H,m),3.52(2H,d,J=7.4Hz), 6.75(1H,s),7.12-7.28(1H,m),7.29-7.33 (1H,m),8.17-8.20(1H,m),1 3.57(1H,br-s). |
| 28 | NMR-D:1.25-1.26(2H,m),1.50-1.52(2H,m),1.60-1.62(4H,m),2.25-2.28(1H,m),3-74(2H,d,J=6.5Hz),6.79(1H, s),7.07-7.12(1H,m),7.33-7.38(1H,m),8.20(1H,d,J=7.0Hz),13.56(1H,brs). m.p.:151-153˚C |

[Table 51]

| Ex | Data |
|---|---|
| 31 | NMR-D:0.88(6H,d,J=6.7Hz),2.00-2.10(1H,m),3.84(2H,d,J=7.3Hz), 6.81(1H,s),7.08-7.12(1H,m),7.44-7.46 (1H,m),8.36(1H,d,J=7.6Hz), 13.57(1H,brs). m.p.:142-144˚C |
| 32 | NMR-D:1.72-1.85(4H,m),1.93-1.97(2H,m),2.63-2.70(1H,m),3.85(2 H,d,J=7.0Hz),6.79(1H,s),7.06-7.11(1H, m),7.31-7.36(1H,m),8.18(1 H,d,J=7.6Hz). m.p.:166-168˚C |
| 37 | NMR-D:0.31-0.35(2H,m),0.45-0.50(2H,m),1.15-1.18(1H,m),3.69(2 H,d,J=6.9Hz),6.79(1H,s),7.08-7.13(1H, m),7.34-7.39(1H,m),8.20(1 H,d,J=7.7Hz),13.54(1H,brs). m.p.:171-173˚C |
| 43 | NMR-D:1.25-1.30(2H,m),1.49-1.52(2H,m),1.60-1.64(4H,m),2.23-2.30(1H,m),2.99(3H,s),3.01(3H,s),3.74 (2H,d,J=7.3Hz),7.03-7.06 (1H,m),7.24-7.30(2H,m),7.41(1H,d,J=7.6Hz). m.p.:51-53˚C |
| 45 | NMR-D:0.84-0.88(6H,m),1.26-1.33(4H,m),1.64-1.68(1H,m),3.43-3.46(2H,m),3.53-3.55(2H,m),3.65-3.70 (6H,m),7.04-7.09(1H,m),7. 26-7.31(2H,m),7.44(1H,d,J=7.6Hz). m.p.:102-104˚C |
| 47 | NMR-D:0.83-0.90(6H,m),1.13-1.20(1H,m),1.32-1.41(1H,m),1.76-1.81(1H,m),3.58-3.72(2H,m),6.79(1H,s), 7.07-7.12(1H,m),7.32-7. 37(1H,m),8.20(1H,d,J=7.6Hz),13.51(1H,brs). m.p.:137-139˚C |
| 49 | NMR-D:0.91(3H,s),0.93(3H,s),1.47-1.52(2H,m),1.5 5-1.62(1H,m), 3.79-3.83(2H,m),6.78(1H,s),7.07-7.12 (1H,m),7.32-7.37(1H,m),8. 19(1H,d,J=7.7Hz),13.53(1H,brs). m.p.:142-144˚C |
| 59 | NMR-D:0.92-1.06(2H,m),1.07-1.21(3H,m),1.52-1.81(6H,m),3.00(6 H,s),3.54(2H,d,J=8.0Hz),7.01(1H,td, J=7.6,1.0Hz),7.07(1H,d,J=7.6 Hz),7.16 (1H,s),7.35(1H,td,J=7.6,1.0Hz),7.57(1H,d,J=7.6Hz). m.p.:80-82˚C |
| 60 | NMR-D:1.21-1.34(2H,m),1.42-1.55(2H,m),1.57-1.70(4H,m),2.25-2.34(1H,m),3.00(3H,s),3.01(3H,s),3.64 (2H,d,J=7.6Hz),7.02(1H,t d,J=7.6,0.8 Hz),7.10(1H,d,J=7.6Hz),7.17(1H,s),7.36(1H,td,J=7.6, 1.2Hz),7.57(1H, d,J=7.6Hz). m.p.:60-62˚C |

[Table 52]

| Ex | Data |
|---|---|
| 61 | NMR-D:0.91-1.06(2H,m),1.07-1.21(3H,m),1.54-1.78(6H,m),2.20-2.30(2H,m),3.54(2H,d,J=7.2Hz),4.03(2H,t, J=8.0Hz),4.30(2H,t,J= 8.0Hz),6.81 (1H,s),7.02(1H,td,J=7.6,1.2Hz),7.06(1H,d,J=7.6Hz), 7.38(1H,td, J=7.6,1.2Hz),8.43(1H,d,J=7.6Hz).<br>m.p.:163-165°C |
| 62 | NMR-D:1.20-1.32(2H,m),1.41-1.55(2H,m),1.56-1.69(4H,m),2.20-2.35(3H,m),3.64(2H,d,J=7.6Hz),4.03(2H,t, J=7.6Hz),4.30(2H,t,J= 7.6 Hz),6.81(1H,s),7.03(1H,td,J=7.6,1.2Hz),7.09(1H,d,J=7.6Hz), 7.38(1H,td, J=7.6,1.6Hz),8.44(1H,d,J=7.6Hz).<br>m.p.:111-113°C |
| 63 | NMR-D:0.87(6H,t,J=7.4Hz),1.24-1.35(4H,m),1.69-1.35(1H,m),3.5 9(2H,d,J=7.6Hz),6.70(1H,s),7.03-7.10 (2H,m),4.41-7.45(1H,m),8.3 4-8.36(1H,m),13.39(1H,brs).<br>m.p.:147-149°C |
| 64 | NMR-D:0.87(6H,t,J=7.4Hz),1.24-1.35(4H,m),1.69-1.76(1H,m),1.8 1-1.93(4H,m),3.46-3.52(4H,m),3.60(2H,d, J=7.5Hz),7.00-7.04(2H, m),7.10(1H,s),7.35-7.39(1H,m),8.04(1H,d,J=7.7Hz).<br>m.p.:71-73°C |
| 65 | NMR-D:0.87(6H,t,J=7.4Hz),1.25-1.33(4H,m),1.71-1.74(1H,m),2.2 2-2.30(2H,m),3.58-3.60(2H,m),4.01-4.05 (2H,m),4.28-4.32(2H,m), 6.82 (1H,s),6.99-7.05(2H,m),7.37-7.41(1H,m),8.44(1H,d,J=7.7H z).<br>m.p.:116-118°C |
| 66 | NMR-D:1.73-1.85(4H,m),1.92-1.99(2H,m),2.22-2.28(2H,m),2.65-2.73(1H,m),3.74-3.75(2H,m),4.01-4.05 (2H,m),4.28-4.31(2H,m),6. 81 (1H,s),7.00-7.08(2H,m),7.35-7.39(1H,m),8.42(1H,d,J=7.7Hz).<br>m.p.:133-135°C |
| 67 | NMR-D:1.72-1.99(10H,m),2.65-2.73(1H,m),3.46-3.52(4H,m),3.75 (2H,d,J=7.2Hz),6.99-7.09(3H,m), 7.34-7.38(1H,m),8.02(1H,d,J=7. 7Hz).<br>m.p.:108-110°C |

Industrial Applicability

The compound of the formula (I) or a salt thereof has a TRPA1 channel activation activity, and can be used as an active ingredient of a pharmaceutical composition for preventing and/or treating constipation-type IBS, atonic constipation and/or functional gastrointestinal disorder, or the like.

Sequence Listing Free Text

In the following sequence listing, the explanations for "human TRPA1 cDNA (sequence number 1)" and "Artificial Sequence (sequence number 2, sequence number 3)" are described. Specifically, nucleotide sequences, which are represented by sequence number 2 and sequence number 3 of sequence listing, are artificially synthesized primer sequences and were used for the cloning of sequence number 1.

## Claims

**1.** A compound of the formula (I) or a salt thereof.

[chem. 19]

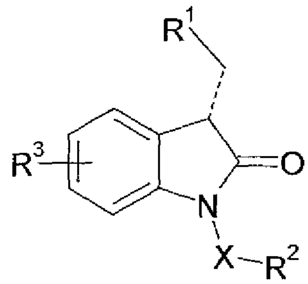

(I)

(wherein,

$R^1$ is $-CO_2H$ or a biological equivalent thereof, $-CO_2-R^0$, $-CON(-R^4)(-R^5)$, -CN, -CO-(nitrogen-containing hetero ring which may be substituted with $-R^0$), or nitrogen-containing hetero ring which may be substituted with $-R^0$,
$R^0$ is $C_{1-6}$ alkyl,
$R^4$ and $R^5$ are the same or different, representing -H, $C_{1-6}$ alkyl, $C_{3-8}$ cycloalkyl, -OH, or $-SO_2-C_{1-6}$ alkyl,
X is $C_{1-10}$ alkylene, or $-(C_{1-10}$ alkylene)-O-,
$R^2$ is (i) hetero ring, aryl, $C_{3-8}$ cycloalkyl or $-CO-R^0$, each of which may be substituted with group(s) selected from $-O-R^0$, $-O-R^{00}$-aryl, $-CO_2-R^0$, $-CON(-R^4)(-R^5)$, -CO-(nitrogen-containing hetero ring which may be substituted with $-R^0$), $-CONHSO_2-R^0$, -CONHOH, $-CO_2H$, $-NO_2$ and -CN, or (ii) -H, or $-R^0$,
$R^{00}$ is a bond or $C_{1-6}$ alkylene,
$R^3$ is -H, $-R^0$, $C_{1-6}$ alkyl which may be substituted with one or more halogens, halogen, $-NO_2$, -CN, or $-O-R^0$,
the dotted line is Z-olefin or E-olefin, or a mixture thereof,

provided that, (a) when $R^1$ is methoxycarbonyl, ethoxycarbonyl, N,N-dimethylaminocarbonyl or N-phenylaminocarbonyl, and $-X-R^2$ is methyl, $R^3$ represents a group other than -H, and (b) when $R^1$ is ethoxycarbonyl, $-CO_2H$ or $-CON(CH_3)_2$, and $-X-R^2$ is benzyl, $R^3$ represents a group other than -H.)

2. The compound or a salt thereof according to claim 1, wherein $R^1$ is $-CO_2H$ or a biological equivalent thereof, $-CO_2-R^0$, $-CON(-R^4)(-R^5)$, -CN, -CO-(nitrogen-containing hetero ring) or nitrogen-containing hetero ring which may be substituted with $-R^0$, $R^4$ and $R^5$ are the same or different, representing -H, or $C_{1-6}$ alkyl, and $R^2$ is (i) hetero ring, aryl, cycloalkyl or $-CO-R^0$, each of which may be substituted with group(s) selected from $-OR^0$,- $O-R^{00}$-aryl, $-CO_2-R^0$, $-CON(-R^4)(-R^5)$ -CO-(nitrogen-containing hetero ring), $-CONHSO_2-R^0$, -CONHOH, $-NO_2$ and -CN, or (ii) -H, or $-R^0$.

3. The compound or a salt thereof according to claim 1, wherein $R^1$ is $-CO_2H$, $-CON(-R^4)(-R^5)$, -CN, -CO-(nitrogen-containing hetero ring which may be substituted with $-R^0$) or nitrogen-containing hetero ring which may be substituted with $-R^0$, $R^2$ is (i) hetero ring, aryl or cycloalkyl, each of which may be substituted with group(s) selected from $-O-R^0$, $-O-R^{00}$-aryl, $-CO_2-R^0$ and $-CO_2H$, or (ii)-H, and $R^3$ is -H, $-R^0$, halogen or $-O-R^0$.

4. The compound or a salt thereof according to claim 3, wherein the dotted line is E-olefin.

5. The compound or a salt thereof according to claim 4, wherein $R^1$ is $-CO_2H$, $-CONH_2$, $-CON(CH_3)_2$ or -CO-(cyclic amino which may be substituted with $-R^0$).

6. The compound or a salt thereof according to claim 5, wherein $R^3$ is -H, -F or -Cl.

7. The compound or a salt thereof according to claim 6, wherein $-X-R^2$ is $C_{4-6}$ alkyl.

8. The compound or a salt thereof according to claim 7, wherein $-X-R^2$ is 2-methylpropan-1-yl, 2-methylbutan-1-yl, 2,2-dimethylpropan-1-yl, 2-ethylbutan-1-yl, 3-methylbutan-1-yl, or 3-methylpentan-1-yl.

9. The compound or a salt thereof according to claim 6, wherein $-X-R^2$ is $C_{3-8}$ cycloalkylmethyl or benzyl in which the benzene ring may be substituted with group(s) selected from the group consisting of $-O-R^0$ and $-CO_2-R^0$.

**10.** The compound or a salt thereof according to claim 9, wherein -X-$R^2$ is cyclopropylmethyl, cyclobutylmethyl, cyclopentylmethyl, cyclohexylmethyl or benzyl

**11.** The compound or a salt thereof according to claim 8 or 10, wherein $R^1$ is -$CO_2H$.

**12.** The compound or a salt thereof according to claim 8 or 10, wherein $R^1$ is -$CONH_2$ or -$CON(CH_3)_2$.

**13.** The compound or a salt thereof according to claim 8 or 10, wherein $R^1$ is pyrrolidin-1-ylcarbonyl, azetidin-1-ylcarbonyl or morpholin-4-ylcarbonyl.

**14.** A compound or a salt thereof, which is
(2E)-[1-(cyelohexylmethyl)-2-oxo-1,2-dihydro-3H-indol-3-ylidene]acetic acid,
(2E)-(1-benzyl-5-fluoro-2-oxo-1,2-dihydro-3H-indol-3-ylidene)acetic acid,
(2E)-(1-benzyl-7-fluoro-2-oxo-1,2-dihydro-3H-indol-3-ylidene)acetic acid,
(2E)-[1-(cyclopentylmethyl)-2-oxo-1,2-dihydro-3H-indol-3-ylidene] acetic acid,
(2E)-(7-fluoro-1-isobutyl-2-oxo-1,2-dihydro-3H-indol-3-ylidene)acetic acid,
(2E)-[1-(cyclopentylmethyl)-7-fluoro-2-oxo-1,2-dihydro-3H-indol-3-ylidene]acetic acid,
(2E)-(7-chloro-1-isobutyl-2-oxo-1,2-dihydro-3H-indol-3-ylidene)acetic acid,
(2E)-[1-(cyclobutylmethyl)-7-fluoro-2-oxo-1,2-dihydro-3H-indol-3-ylidene]acetic acid,
(2E)-[1-(cyclopropylmethyl)-7-fluoro-2-oxo-1,2-dihydro-3H-indol-3-ylidene]acetic acid,
(2E)-2-[1-(cydopentylmethyl)-7-fluoro-2-oxo-1,2,dihydro,3H-indol-3-ylidene]-N,N-dimethylacetamide,
(3E)-1-(2-ethyl butyl)-7-fluoro-3-(2-morpholin-4-yl-2-oxoethylidene)-1,3-dihydro-2H-indol-2-one,
(2E)- {7-fluoro-1-[(2S)-2-methylbutyl]-2-oxo-1,2-d]hydro-3H-indol-3-ylidene}acetic acid,
(2E)-[7-fluoro-1-(3-methylbutyl)-2-oxo-1,2-dihydro-3H-indol-3-ylidene]acetic acid,
(2E)-2-[1-(cydohexylmethyl)-2-oxo-1,2-dinhydro-3H-indol-3-ylidene]-N,N-dimethylacetamide,
(2E)-2-[1-(cyclopentylmethyl)-2-oxo-1,2-dlhydro-3H-indol-3-ylidene]-N,N-dimethylacetamide,
(3E)-3-(2-azetidin-1-yl-2-oxoethylidene)-1-(cyclohexylmethyl)-1,3-dihydro-2H-indol-2-one,
(3E)-3-(2-azetidin-1-yl-2-oxoethylidene)-1-(cyclopentylmethyl)-1,3-dihydro-2H-indol-2-one,
(2E)-[1-(2-ethylbutyl)-2-oxo-1,2-dihydro-3H-indol-3-ylidene]acetic acid,
(3E)-1-(2-ethylbutyl)-3-(2-oxo-2-pyrrolidin-1-ylethylidene)-1,3-dihydro-2H-indol-2-one,
(3E)-3-(2-azetidin-1-yl-2-oxoethylidene)-1-(2-ethylbutyl)-1,3-dihydro-2H-indol-2-one,
(3E)-3-(2-azetidin-1-yl-2-oxoethylidene)-1-(cyclobutylmethyl)-1,3-dihydro-2H-indol-2-one, or,
(3E)-1-(cyclobutylmethyl)-3-(2-oxo-2-pyrrolidin-1-ylethylidene)-1,3-dihydro-2H-indol-2-one,
or a salt thereof, among the compound or a salt thereof according to claim 1.

**15.** A pharmaceutical composition comprising the compound or a salt thereof according to claim 1, and a pharmaceutically acceptable excipient.

**16.** A pharmaceutical composition for treating constipation-type irritable bowel syndrome, atonic constipation or functional gastrointestinal disorder comprising the compound or a salt thereof according to claim 1.

**17.** Use of the compound or a salt thereof according to claim 1 for the manufacture of a pharmaceutical composition for treating constipation-type irritable bowel syndrome, atonic constipation or functional gastrointestinal disorder.

**18.** The compound or a salt thereof according to claim 1 for use in the treatment of constipation-type irritable bowel syndrome, atonic constipation or functional gastrointestinal disorder.

**19.** A method for treating constipation-type irritable bowel syndrome, atonic constipation or functional gastrointestinal disorder, comprising administering an effective amount of the compound or a salt thereof according to claim 1 to a subject.

**INTERNATIONAL SEARCH REPORT**

International application No.
PCT/JP2009/056431

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D209/34, A61K31/4045, A61K31/405, A61K31/4178, A61K31/427, A61K31/4439, A61K31/5377, A61P1/04, A61P1/14, C07D401/06, C07D403/06, C07D405/06, C07D417/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>A | BRAUD, E., Potential Inhibitors of Angiogenesis. Part I: 3-(Imidazol-4(5)-ylmethylene)indolin-2-ones, Journal of Enzyme Inhibition and Medicinal Chemistry, 2003, Vol.18, No.3, p.243-252, page 249, Fig. 3, compounds 20 to 40, 41 to 50, 51; page 250, table 1, compounds 35 to 40, 41, 42, 44 to 47, 50 | 1-4,15,18<br>5-14,16,17 |
| X<br>A | ANDREANI, A., Antitumor Activity of New Substituted 3-(5-Imidazo[2,1-b] thiazolylmethylene)-2-indolinones and Study of Their Effect on the Cell Cycle, Journal of Medicinal Chemistry, 2005, Vol.48, No.17, p.5604-5607, page 5605, Scheme 1, compounds 16 to 28; table 1, compound 24 | 1-4,15,18<br>5-14,16,17 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>11 May, 2009 (11.05.09) | Date of mailing of the international search report<br>26 May, 2009 (26.05.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/056431

C (Continuation). DOCUMENTS CONSIDERED TO BE RELEVANT

| Category* | Citation of document, with indication, where appropriate, of the relevant passages | Relevant to claim No. |
|---|---|---|
| X<br>A | WO 2007/062399 A2 (UNIV TEXAS SYSTEM),<br>31 May, 2007 (31.05.07),<br>Page 24, compound K046<br>& EP 1956910 A2 | 1-3,15,18<br>4-14,16,17 |
| X<br>A | WO 2006/105796 A1 (LEO PHARMA AS),<br>12 October, 2006 (12.10.06),<br>Page 74, example 23; page 75, example 24;<br>page 77, example 28; page 80, example 34;<br>page 83, examples 39, 40; page 84, example 42;<br>page 86, example 44<br>(Family: none) | 1-3,15,18<br>4-14,16,17 |
| X<br>A | JP 2003-534341 A (SUGEN INC.),<br>18 November, 2003 (18.11.03),<br>Page 100, example 1; page 101, examples 2, 3;<br>page 102, example 4; page 103, Par. No. [0219];<br>page 107, example 12; page 108, example 14<br>& WO 2001/90103 A2 & US 2002/037878 A1<br>& US 6482848 B2 & EP 1283835 A2<br>& US 2003/083363 A1 & US 6716870 B2<br>& US 2005/107340 A1 & US 7112603 B2 | 1-3,15,18<br>4-14,16,17 |
| X<br>A | US 3632587 A (WYETH & BROTHER LTD. JOHN),<br>04 January, 1972 (04.01.72),<br>Column 2, lines 8 to 14; column 4, examples 2,<br>3<br>& GB 1239553 A | 1,2,15,18<br>3-14,16,17 |
| X<br>A | MORI, M., Preparation and synthetic use of the<br>zerovalent nickel complex by electrochemical<br>reduction, Tetrahedron Letters, 1980, Vol.21,<br>No.7, p.631-634, page 633, table 2,<br>compounds b, c | 1-4,18<br>5-17 |
| X<br>A | SUGASAWA, S., Synthesis of dl-esermethole,<br>Chemical & Pharmaceutical Bulletin, 1958,<br>Vol.6, p.194-200, page 196, Chart 2,<br>compound (XV) | 1-4,18<br>5-17 |
| X<br>A | JP 2007-047770 A (FUJI FILM CO., LTD.),<br>22 February, 2007 (22.02.07),<br>Pages 11 to 14, compounds (7) to (12), (21),<br>(26), (35)<br>& EP 1744212 A2 & US 2007/015087 A1<br>& CN 1896873 A | 1-3,18<br>4-17 |
| X<br>A | GABRIELE, B., Stereoselective synthesis of<br>(E)-3-(methoxycarbonyl)<br>methylene-1,3- dihydroindol-2-ones by<br>palladium-catalyzed oxidative carbonylation of<br>2-ethynylanilines, European Journal of Organic<br>Chemistry, 2001, No.24, p.4607-4613, page 4607,<br>compounds 2b, 2c | 1,2,18<br>3-17 |

Form PCT/ISA/210 (continuation of second sheet) (April 2007)

INTERNATIONAL SEARCH REPORT

International application No. PCT/JP2009/056431

C (Continuation). DOCUMENTS CONSIDERED TO BE RELEVANT

| Category* | Citation of document, with indication, where appropriate, of the relevant passages | Relevant to claim No. |
|---|---|---|
| X<br>A | OKADA, K., Diels-Alder reaction of 3-(methoxycarbonylmethylene)-2-oxoindoline derivatives with unsymmetrical butadienes, Chemistry Letters, 1979, No.3, p.213-216, page 213, compound (1b) | 1,2,18<br>3-17 |
| X<br>A | GALLIFORD, C. V., A highly diastereoselective, catalytic three-component assembly reaction for the synthesis of spiropyrrolidinyloxindoles, Chemical Communications(Cambridge, United Kingdom), 2007, No.6, p.631-633, page 631, compound 3 | 1,2,18<br>3-17 |
| P,X<br>P,A | SHIMAZAWA, R., Design and synthesis of N-alkyl oxindolylidene acetic acids as a new class of potent Cdc25A inhibitors, Bioorganic & Medicinal Chemistry Letters, 2008.06.01, Vol.18, No.11, p.3350-3353 | 1-7,15,18<br>8-14,16,17 |
| A | WO 2006/115135 A1 (ASTELLAS PHARMA INC.), 02 November, 2006 (02.11.06), Page 22, lines 1 to 2 & EP 1872795 A1 & JP 2007-514619 A | 1-18 |
| A | WO 2008/019357 A2 (MICROBIA INC.), 14 February, 2008 (14.02.08), Page 17, lines 27 to 29; page 79, line 20; Claims 356, 390, 391 (Family: none) | 1-18 |
| A | MACPHERSON, L. J., Noxious compounds activate TRPA1 ion channels through covalent modification of cysteines, Nature, 2007, Vol.445, No.7127, p.541-545, Table 1 TRPA1 agonist SC | 1-18 |
| P,A | WO 2008/044660 A1 (ASTELLAS PHARMA INC.), 17 April, 2008 (17.04.08), (Family: none) | 1-18 |

Form PCT/ISA/210 (continuation of second sheet) (April 2007)

**INTERNATIONAL SEARCH REPORT**

International application No.
PCT/JP2009/056431

Continuation of A. CLASSIFICATION OF SUBJECT MATTER
(International Patent Classification (IPC))

*C07D209/34*(2006.01)i, *A61K31/4045*(2006.01)i, *A61K31/405*(2006.01)i, *A61K31/4178*(2006.01)i, *A61K31/427*(2006.01)i, *A61K31/4439*(2006.01)i, *A61K31/5377*(2006.01)i, *A61P1/04*(2006.01)i, *A61P1/14*(2006.01)i, *C07D401/06*(2006.01)i, *C07D403/06*(2006.01)i, *C07D405/06*(2006.01)i, *C07D417/06*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2009/056431

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 19
because they relate to subject matter not required to be searched by this Authority, namely:
Claim 19 pertains to methods for treatment of the human body by therapy and thus relates to a subject matter which this International Searching Authority is not required, under the provisions of Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.
PCT/JP2009/056431

<Subject of search>

The term "a biological equivalent" recited in claims 1 and 2 makes the types of the structure of the compound to be included within the scope of claims 1 and 2 unclear. Even though the statements of the description are taken into consideration, it cannot be considered that the term is defined clearly, although examples of the term are mentioned in Paragraph No. [0036].

Therefore, claims 1, 2 and 15-18 and the description do not comply with the prescribed requirements in such an extent that a meaningful international search can be made. Such being the case, the search was made mainly on the parts which are supported by and disclosed in the description.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200382265 A
- US 3428649 A
- WO 200819357 A
- WO 200456769 A


### Non-patent literature cited in the description


- Textbook of Gastroenterolorogy
- *Gastroenterology,* 2006, vol. 130, 34-43
- *Clinical Gastroenterology and Hepatology,* 2005, vol. 3, 349-357
- *Nature,* 2007, vol. 445, 541-545
- *Nature,* 2007, vol. 445, 491-492
- *Journal of Chemical Research,* 2005, vol. 1, 62-66
- *Journal of the American Chemical Society,* 1994, vol. 116, 9480-9486
- *Tetrahedron,* 1967, vol. 23, 901-917
- *Tetrahedron,* 2002, vol. 58, 7221-7231
- *Gazzetta Chimica Italiana,* 1968, vol. 98, 344-357
- *Bioorganic and Medicinal Chemistry Letters,* 2008, vol. 18, 3350-3353
- *Prog. Med.,* 1985, vol. 5, 2157-2161
- Pharmaceutical Research and Development. Drug Design. Hirokawa Publishing Company, 1990, vol. 7, 163-198
- Green's Protective Groups in Organic Synthesis. 2006
- **Toshio Kuroki ; Namho Hue ; Kazuhiro Chida.** Cultivation Cell Experimental Methods for Molecular Biology Study. Yodosha, 1995
- **Crespi et al.** *Analytical Biochemistry,* 1997, vol. 248, 188-190

SEQUENCE LISTING

<110> ASTELLAS PHARMA INC.

<120> INDOLINONE COMPOUND

<130> 21912EP

<140> 09 729 006.8
<141> 2009-03-30

<150> JP 2008-0095040
<151> 2008-04-01

<160> 4

<170> PatentIn version 3.5

<210> 1
<211> 3360
<212> DNA
<213> Homo sapiens
<220>
<221> CDS
<222> (1)..(3360)

<400> 1
atg aag tgc agc ctg agg aag atg tgg cgc cct gga gaa aag aag gag 48
Met Lys Cys Ser Leu Arg Lys Met Trp Arg Pro Gly Glu Lys Lys Glu
1 5 10 15

ccc cag ggc gtt gtc tat gag gat gtg ccg gac gac acg gag gat ttc 96
Pro Gln Gly Val Val Tyr Glu Asp Val Pro Asp Asp Thr Glu Asp Phe
20 25 30

aag gaa tcg ctt aag gtg gtt ttt gaa gga agt gca tat gga tta caa 144
Lys Glu Ser Leu Lys Val Val Phe Glu Gly Ser Ala Tyr Gly Leu Gln
35 40 45

aac ttt aat aag caa aag aaa tta aaa aca tgt gac gat atg gac acc 192
Asn Phe Asn Lys Gln Lys Lys Leu Lys Thr Cys Asp Asp Met Asp Thr
50 55 60

ttc ttc ttg cat tat gct gca gca gaa ggc caa att gag cta atg gag 240
Phe Phe Leu His Tyr Ala Ala Ala Glu Gly Gln Ile Glu Leu Met Glu
65 70 75 80

aag atc acc aga gat tcc tct ttg gaa gtg ctg cat gaa atg gat gat 288
Lys Ile Thr Arg Asp Ser Ser Leu Glu Val Leu His Glu Met Asp Asp
85 90 95

tat gga aat acc cct ctg cat tgt gct gta gaa aaa aac caa att gaa 336
Tyr Gly Asn Thr Pro Leu His Cys Ala Val Glu Lys Asn Gln Ile Glu
100 105 110

agc gtt aag ttt ctt ctc agc aga gga gca aac cca aac ctc cga aac 384

```
Ser Val Lys Phe Leu Leu Ser Arg Gly Ala Asn Pro Asn Leu Arg Asn
        115                 120                 125

ttc aac atg atg gct cct ctc cac ata gct gtg cag ggc atg aat aat      432
Phe Asn Met Met Ala Pro Leu His Ile Ala Val Gln Gly Met Asn Asn
    130                 135                 140

gag gtg atg aag gtc ttg ctt gag cat aga act att gat gtt aat ttg      480
Glu Val Met Lys Val Leu Leu Glu His Arg Thr Ile Asp Val Asn Leu
145                 150                 155                 160

gaa gga gaa aat gga aac aca gct gtg atc att gcg tgc acc aca aat      528
Glu Gly Glu Asn Gly Asn Thr Ala Val Ile Ile Ala Cys Thr Thr Asn
                165                 170                 175

aat agc gaa gca ttg cag att ttg ctt aac aaa gga gct aag cca tgt      576
Asn Ser Glu Ala Leu Gln Ile Leu Leu Asn Lys Gly Ala Lys Pro Cys
            180                 185                 190

aaa tca aat aaa tgg gga tgt ttc cct att cac caa gct gca ttt tca      624
Lys Ser Asn Lys Trp Gly Cys Phe Pro Ile His Gln Ala Ala Phe Ser
        195                 200                 205

ggt tcc aaa gaa tgc atg gaa ata ata cta agg ttt ggt gaa gag cat      672
Gly Ser Lys Glu Cys Met Glu Ile Ile Leu Arg Phe Gly Glu Glu His
    210                 215                 220

ggg tac agt aga cag ttg cac att aac ttt atg aat aat ggg aaa gcc      720
Gly Tyr Ser Arg Gln Leu His Ile Asn Phe Met Asn Asn Gly Lys Ala
225                 230                 235                 240

acc cct ctc cac ctg gct gtg caa aat ggt gac ttg gaa atg atc aaa      768
Thr Pro Leu His Leu Ala Val Gln Asn Gly Asp Leu Glu Met Ile Lys
                245                 250                 255

atg tgc ctg gac aat ggt gca caa ata gac cca gtg gag aag gga agg      816
Met Cys Leu Asp Asn Gly Ala Gln Ile Asp Pro Val Glu Lys Gly Arg
            260                 265                 270

tgc aca gcc att cat ttt gct gcc acc cag gga gcc act gag att gtt      864
Cys Thr Ala Ile His Phe Ala Ala Thr Gln Gly Ala Thr Glu Ile Val
        275                 280                 285

aaa ctg atg ata tcg tcc tat tct ggt agc gtg gat att gtt aac aca      912
Lys Leu Met Ile Ser Ser Tyr Ser Gly Ser Val Asp Ile Val Asn Thr
    290                 295                 300

acc gat gga tgt cat gag acc atg ctt cac aga gct tca ttg ttt gat      960
Thr Asp Gly Cys His Glu Thr Met Leu His Arg Ala Ser Leu Phe Asp
305                 310                 315                 320

cac cat gag cta gca gac tat tta att tca gtg gga gca gat att aat     1008
His His Glu Leu Ala Asp Tyr Leu Ile Ser Val Gly Ala Asp Ile Asn
                325                 330                 335

aag atc gat tct gaa gga cgc tct cca ctt ata tta gca act gct tct     1056
Lys Ile Asp Ser Glu Gly Arg Ser Pro Leu Ile Leu Ala Thr Ala Ser
            340                 345                 350
```

```
gca tct tgg aat att gta aat ttg cta ctc tct aaa ggt gcc caa gta      1104
Ala Ser Trp Asn Ile Val Asn Leu Leu Leu Ser Lys Gly Ala Gln Val
        355                 360                 365

gac ata aaa gat aat ttt gga cgt aat ttt ctg cat tta act gta cag      1152
Asp Ile Lys Asp Asn Phe Gly Arg Asn Phe Leu His Leu Thr Val Gln
    370                 375                 380

caa cct tat gga tta aaa aat ctg cga cct gaa ttt atg cag atg caa      1200
Gln Pro Tyr Gly Leu Lys Asn Leu Arg Pro Glu Phe Met Gln Met Gln
385                 390                 395                 400

cag atc aaa gag ctg gta atg gat gaa gac aac gat ggg tgt act cct      1248
Gln Ile Lys Glu Leu Val Met Asp Glu Asp Asn Asp Gly Cys Thr Pro
                405                 410                 415

cta cat tat gca tgt aga cag ggg ggc cct ggt tct gta aat aac cta      1296
Leu His Tyr Ala Cys Arg Gln Gly Gly Pro Gly Ser Val Asn Asn Leu
            420                 425                 430

ctt ggc ttt aat gtg tcc att cat tcc aaa agc aaa gat aag aaa tca      1344
Leu Gly Phe Asn Val Ser Ile His Ser Lys Ser Lys Asp Lys Lys Ser
        435                 440                 445

cct ctg cat ttt gca gcc agt tat ggg cgt atc aat acc tgt cag agg      1392
Pro Leu His Phe Ala Ala Ser Tyr Gly Arg Ile Asn Thr Cys Gln Arg
    450                 455                 460

ctc cta caa gac ata agt gat acg agg ctt ctg aat gaa ggt gac ctt      1440
Leu Leu Gln Asp Ile Ser Asp Thr Arg Leu Leu Asn Glu Gly Asp Leu
465                 470                 475                 480

cat gga atg act cct ctc cat ctg gca gca aag aat gga cat gat aaa      1488
His Gly Met Thr Pro Leu His Leu Ala Ala Lys Asn Gly His Asp Lys
                485                 490                 495

gta gtt cag ctt ctt ctg aaa aaa ggt gca ttg ttt ctc agt gac cac      1536
Val Val Gln Leu Leu Leu Lys Lys Gly Ala Leu Phe Leu Ser Asp His
            500                 505                 510

aat ggc tgg aca gct ttg cat cat gcg tcc atg ggc ggg tac act cag      1584
Asn Gly Trp Thr Ala Leu His His Ala Ser Met Gly Gly Tyr Thr Gln
        515                 520                 525

acc atg aag gtc att ctt gat act aat ttg aag tgc aca gat cgc ttg      1632
Thr Met Lys Val Ile Leu Asp Thr Asn Leu Lys Cys Thr Asp Arg Leu
    530                 535                 540

gat gaa gac ggg aac act gca ctt cac ttt gct gca agg gaa ggc cac      1680
Asp Glu Asp Gly Asn Thr Ala Leu His Phe Ala Ala Arg Glu Gly His
545                 550                 555                 560

gcc aaa gcc gtt gcg ctt ctt ctg agc cac aat gct gac ata gtc ctg      1728
Ala Lys Ala Val Ala Leu Leu Leu Ser His Asn Ala Asp Ile Val Leu
                565                 570                 575

aac aag cag cag gcc tcc ttt ttg cac ctt gca ctt cac aat aag agg      1776
```

```
Asn Lys Gln Gln Ala Ser Phe Leu His Leu Ala Leu His Asn Lys Arg
            580                 585                 590

aag gag gtt gtt ctt acg atc atc agg agc aaa aga tgg gat gaa tgt      1824
Lys Glu Val Val Leu Thr Ile Ile Arg Ser Lys Arg Trp Asp Glu Cys
        595                 600                 605

ctt aag att ttc agt cat aat tct cca ggc aat aaa tgt cca att aca      1872
Leu Lys Ile Phe Ser His Asn Ser Pro Gly Asn Lys Cys Pro Ile Thr
    610                 615                 620

gaa atg ata gaa tac ctc cct gaa tgc atg aag gta ctt tta gat ttc      1920
Glu Met Ile Glu Tyr Leu Pro Glu Cys Met Lys Val Leu Leu Asp Phe
625                 630                 635                 640

tgc atg ttg cat tcc aca gaa gac aag tcc tgc cga gac tat tat atc      1968
Cys Met Leu His Ser Thr Glu Asp Lys Ser Cys Arg Asp Tyr Tyr Ile
                645                 650                 655

gag tat aat ttc aaa tat ctt caa tgt cca tta gaa ttc acc aaa aaa      2016
Glu Tyr Asn Phe Lys Tyr Leu Gln Cys Pro Leu Glu Phe Thr Lys Lys
            660                 665                 670

aca cct aca cag gat gtt ata tat gaa ccg ctt aca gcc ctc aac gca      2064
Thr Pro Thr Gln Asp Val Ile Tyr Glu Pro Leu Thr Ala Leu Asn Ala
        675                 680                 685

atg gta caa aat aac cgc ata gag ctt ctc aat cat cct gtg tgt aaa      2112
Met Val Gln Asn Asn Arg Ile Glu Leu Leu Asn His Pro Val Cys Lys
    690                 695                 700

gaa tat tta ctc atg aaa tgg ttg gct tat gga ttt aga gct cat atg      2160
Glu Tyr Leu Leu Met Lys Trp Leu Ala Tyr Gly Phe Arg Ala His Met
705                 710                 715                 720

atg aat tta gga tct tac tgt ctt ggt ctc ata cct atg acc att ctc      2208
Met Asn Leu Gly Ser Tyr Cys Leu Gly Leu Ile Pro Met Thr Ile Leu
                725                 730                 735

gtt gtc aat ata aaa cca gga atg gct ttc aac tca act ggc atc atc      2256
Val Val Asn Ile Lys Pro Gly Met Ala Phe Asn Ser Thr Gly Ile Ile
            740                 745                 750

aat gaa act agt gat cat tca gaa ata cta gat acc acg aat tca tat      2304
Asn Glu Thr Ser Asp His Ser Glu Ile Leu Asp Thr Thr Asn Ser Tyr
        755                 760                 765

cta ata aaa act tgt atg att tta gtg ttt tta tca agt ata ttt ggg      2352
Leu Ile Lys Thr Cys Met Ile Leu Val Phe Leu Ser Ser Ile Phe Gly
    770                 775                 780

tat tgc aaa gaa gcg ggg caa att ttc caa cag aaa agg aat tat ttt      2400
Tyr Cys Lys Glu Ala Gly Gln Ile Phe Gln Gln Lys Arg Asn Tyr Phe
785                 790                 795                 800

atg gat ata agc aat gtt ctt gaa tgg att atc tac acg acg ggc atc      2448
Met Asp Ile Ser Asn Val Leu Glu Trp Ile Ile Tyr Thr Thr Gly Ile
                805                 810                 815
```

```
att ttt gtg ctg ccc ttg ttt gtt gaa ata cca gct cat ctg cag tgg     2496
Ile Phe Val Leu Pro Leu Phe Val Glu Ile Pro Ala His Leu Gln Trp
            820                 825                 830

caa tgt gga gca att gct gtt tac ttc tat tgg atg aat ttc tta ttg     2544
Gln Cys Gly Ala Ile Ala Val Tyr Phe Tyr Trp Met Asn Phe Leu Leu
        835                 840                 845

tat ctt caa aga ttt gaa aat tgt gga att ttt att gtt atg ttg gag     2592
Tyr Leu Gln Arg Phe Glu Asn Cys Gly Ile Phe Ile Val Met Leu Glu
    850                 855                 860

gta att ttg aaa act ttg ttg agg tct aca gtt gta ttt atc ttc ctt     2640
Val Ile Leu Lys Thr Leu Leu Arg Ser Thr Val Val Phe Ile Phe Leu
865                 870                 875                 880

ctt ctg gct ttt gga ctc agc ttt tac atc ctc ctg aat tta cag gat     2688
Leu Leu Ala Phe Gly Leu Ser Phe Tyr Ile Leu Leu Asn Leu Gln Asp
                885                 890                 895

ccc ttc agc tct cca ttg ctt tct ata atc cag acc ttc agc atg atg     2736
Pro Phe Ser Ser Pro Leu Leu Ser Ile Ile Gln Thr Phe Ser Met Met
            900                 905                 910

cta gga gat atc aat tat cga gag tcc ttc cta gaa cca tat ctg aga     2784
Leu Gly Asp Ile Asn Tyr Arg Glu Ser Phe Leu Glu Pro Tyr Leu Arg
        915                 920                 925

aat gaa ttg gca cat cca gtt ctg tcc ttt gca caa ctt gtt tcc ttc     2832
Asn Glu Leu Ala His Pro Val Leu Ser Phe Ala Gln Leu Val Ser Phe
    930                 935                 940

aca ata ttt gtc cca att gtc ctc atg aat tta ctt att ggt ttg gca     2880
Thr Ile Phe Val Pro Ile Val Leu Met Asn Leu Leu Ile Gly Leu Ala
945                 950                 955                 960

gtt ggc gac att gct gag gtc cag aaa cat gca tca ttg aag agg ata     2928
Val Gly Asp Ile Ala Glu Val Gln Lys His Ala Ser Leu Lys Arg Ile
                965                 970                 975

gct atg cag gtg gaa ctt cat acc agc tta gag aag aag ctg cca ctt     2976
Ala Met Gln Val Glu Leu His Thr Ser Leu Glu Lys Lys Leu Pro Leu
            980                 985                 990

tgg ttt cta cgc aaa gtg gat cag  aaa tcc acc atc gtg  tat ccc aac   3024
Trp Phe Leu Arg Lys Val Asp Gln  Lys Ser Thr Ile Val  Tyr Pro Asn
        995                 1000                1005

aaa ccc  aga tct ggt ggg atg  tta ttc cat ata ttc  tgt ttt tta     3069
Lys Pro  Arg Ser Gly Gly Met  Leu Phe His Ile Phe  Cys Phe Leu
    1010                 1015                 1020

ttt tgc  act ggg gaa ata aga  caa gaa ata cca aat  gct gat aaa     3114
Phe Cys  Thr Gly Glu Ile Arg  Gln Glu Ile Pro Asn  Ala Asp Lys
    1025                 1030                 1035

tct tta  gaa atg gaa ata tta  aag cag aaa tac cgg  ctg aag gat     3159
```

```
Ser Leu  Glu Met Glu Ile Leu  Lys Gln Lys Tyr Arg  Leu Lys Asp
    1040                 1045                 1050

ctt act  ttt ctc ctg gaa aaa  cag cat gag ctc att  aaa ctg atc      3204
Leu Thr  Phe Leu Leu Glu Lys  Gln His Glu Leu Ile  Lys Leu Ile
    1055                 1060                 1065

att cag  aag atg gag atc atc  tct gag aca gag gat  gat gat agc      3249
Ile Gln  Lys Met Glu Ile Ile  Ser Glu Thr Glu Asp  Asp Asp Ser
    1070                 1075                 1080

cat tgt  tct ttt caa gac agg  ttt aag aaa gag cag  atg gaa caa      3294
His Cys  Ser Phe Gln Asp Arg  Phe Lys Lys Glu Gln  Met Glu Gln
    1085                 1090                 1095

agg aat  agc aga tgg aat act  gtg ttg aga gca gtc  aag gca aaa      3339
Arg Asn  Ser Arg Trp Asn Thr  Val Leu Arg Ala Val  Lys Ala Lys
    1100                 1105                 1110

aca cac  cat ctt gag cct tag                                        3360
Thr His  His Leu Glu Pro
    1115


<210>  2
<211>  1119
<212>  PRT
<213>  Homo sapiens

<400>  2
Met Lys Cys Ser Leu Arg Lys Met Trp Arg Pro Gly Glu Lys Lys Glu
1               5                   10                  15

Pro Gln Gly Val Val Tyr Glu Asp Val Pro Asp Asp Thr Glu Asp Phe
            20                  25                  30

Lys Glu Ser Leu Lys Val Val Phe Glu Gly Ser Ala Tyr Gly Leu Gln
        35                  40                  45

Asn Phe Asn Lys Gln Lys Lys Leu Lys Thr Cys Asp Asp Met Asp Thr
    50                  55                  60

Phe Phe Leu His Tyr Ala Ala Ala Glu Gly Gln Ile Glu Leu Met Glu
65                  70                  75                  80

Lys Ile Thr Arg Asp Ser Ser Leu Glu Val Leu His Glu Met Asp Asp
                85                  90                  95

Tyr Gly Asn Thr Pro Leu His Cys Ala Val Glu Lys Asn Gln Ile Glu
            100                 105                 110

Ser Val Lys Phe Leu Leu Ser Arg Gly Ala Asn Pro Asn Leu Arg Asn
        115                 120                 125

Phe Asn Met Met Ala Pro Leu His Ile Ala Val Gln Gly Met Asn Asn
    130                 135                 140
```

```
Glu Val Met Lys Val Leu Leu Glu His Arg Thr Ile Asp Val Asn Leu
145                 150                 155                 160

Glu Gly Glu Asn Gly Asn Thr Ala Val Ile Ile Ala Cys Thr Thr Asn
                165                 170                 175

Asn Ser Glu Ala Leu Gln Ile Leu Leu Asn Lys Gly Ala Lys Pro Cys
            180                 185                 190

Lys Ser Asn Lys Trp Gly Cys Phe Pro Ile His Gln Ala Ala Phe Ser
        195                 200                 205

Gly Ser Lys Glu Cys Met Glu Ile Ile Leu Arg Phe Gly Glu Glu His
    210                 215                 220

Gly Tyr Ser Arg Gln Leu His Ile Asn Phe Met Asn Asn Gly Lys Ala
225                 230                 235                 240

Thr Pro Leu His Leu Ala Val Gln Asn Gly Asp Leu Glu Met Ile Lys
                245                 250                 255

Met Cys Leu Asp Asn Gly Ala Gln Ile Asp Pro Val Glu Lys Gly Arg
            260                 265                 270

Cys Thr Ala Ile His Phe Ala Ala Thr Gln Gly Ala Thr Glu Ile Val
        275                 280                 285

Lys Leu Met Ile Ser Ser Tyr Ser Gly Ser Val Asp Ile Val Asn Thr
    290                 295                 300

Thr Asp Gly Cys His Glu Thr Met Leu His Arg Ala Ser Leu Phe Asp
305                 310                 315                 320

His His Glu Leu Ala Asp Tyr Leu Ile Ser Val Gly Ala Asp Ile Asn
                325                 330                 335

Lys Ile Asp Ser Glu Gly Arg Ser Pro Leu Ile Leu Ala Thr Ala Ser
            340                 345                 350

Ala Ser Trp Asn Ile Val Asn Leu Leu Leu Ser Lys Gly Ala Gln Val
        355                 360                 365

Asp Ile Lys Asp Asn Phe Gly Arg Asn Phe Leu His Leu Thr Val Gln
    370                 375                 380

Gln Pro Tyr Gly Leu Lys Asn Leu Arg Pro Glu Phe Met Gln Met Gln
385                 390                 395                 400

Gln Ile Lys Glu Leu Val Met Asp Glu Asp Asn Asp Gly Cys Thr Pro
                405                 410                 415

Leu His Tyr Ala Cys Arg Gln Gly Gly Pro Gly Ser Val Asn Asn Leu
            420                 425                 430

Leu Gly Phe Asn Val Ser Ile His Ser Lys Ser Lys Asp Lys Lys Ser
        435                 440                 445
```

```
Pro Leu His Phe Ala Ala Ser Tyr Gly Arg Ile Asn Thr Cys Gln Arg
    450                 455                 460

Leu Leu Gln Asp Ile Ser Asp Thr Arg Leu Leu Asn Glu Gly Asp Leu
465                 470                 475                 480

His Gly Met Thr Pro Leu His Leu Ala Ala Lys Asn Gly His Asp Lys
                485                 490                 495

Val Val Gln Leu Leu Leu Lys Lys Gly Ala Leu Phe Leu Ser Asp His
            500                 505                 510

Asn Gly Trp Thr Ala Leu His His Ala Ser Met Gly Gly Tyr Thr Gln
        515                 520                 525

Thr Met Lys Val Ile Leu Asp Thr Asn Leu Lys Cys Thr Asp Arg Leu
    530                 535                 540

Asp Glu Asp Gly Asn Thr Ala Leu His Phe Ala Ala Arg Glu Gly His
545                 550                 555                 560

Ala Lys Ala Val Ala Leu Leu Leu Ser His Asn Ala Asp Ile Val Leu
                565                 570                 575

Asn Lys Gln Gln Ala Ser Phe Leu His Leu Ala Leu His Asn Lys Arg
            580                 585                 590

Lys Glu Val Val Leu Thr Ile Ile Arg Ser Lys Arg Trp Asp Glu Cys
        595                 600                 605

Leu Lys Ile Phe Ser His Asn Ser Pro Gly Asn Lys Cys Pro Ile Thr
    610                 615                 620

Glu Met Ile Glu Tyr Leu Pro Glu Cys Met Lys Val Leu Leu Asp Phe
625                 630                 635                 640

Cys Met Leu His Ser Thr Glu Asp Lys Ser Cys Arg Asp Tyr Tyr Ile
                645                 650                 655

Glu Tyr Asn Phe Lys Tyr Leu Gln Cys Pro Leu Glu Phe Thr Lys Lys
            660                 665                 670

Thr Pro Thr Gln Asp Val Ile Tyr Glu Pro Leu Thr Ala Leu Asn Ala
        675                 680                 685

Met Val Gln Asn Asn Arg Ile Glu Leu Leu Asn His Pro Val Cys Lys
    690                 695                 700

Glu Tyr Leu Leu Met Lys Trp Leu Ala Tyr Gly Phe Arg Ala His Met
705                 710                 715                 720

Met Asn Leu Gly Ser Tyr Cys Leu Gly Leu Ile Pro Met Thr Ile Leu
                725                 730                 735

Val Val Asn Ile Lys Pro Gly Met Ala Phe Asn Ser Thr Gly Ile Ile
            740                 745                 750
```

```
Asn Glu Thr Ser Asp His Ser Glu Ile Leu Asp Thr Thr Asn Ser Tyr
        755                 760                 765

Leu Ile Lys Thr Cys Met Ile Leu Val Phe Leu Ser Ser Ile Phe Gly
    770                 775                 780

Tyr Cys Lys Glu Ala Gly Gln Ile Phe Gln Gln Lys Arg Asn Tyr Phe
785                 790                 795                 800

Met Asp Ile Ser Asn Val Leu Glu Trp Ile Ile Tyr Thr Thr Gly Ile
                805                 810                 815

Ile Phe Val Leu Pro Leu Phe Val Glu Ile Pro Ala His Leu Gln Trp
            820                 825                 830

Gln Cys Gly Ala Ile Ala Val Tyr Phe Tyr Trp Met Asn Phe Leu Leu
        835                 840                 845

Tyr Leu Gln Arg Phe Glu Asn Cys Gly Ile Phe Ile Val Met Leu Glu
    850                 855                 860

Val Ile Leu Lys Thr Leu Leu Arg Ser Thr Val Val Phe Ile Phe Leu
865                 870                 875                 880

Leu Leu Ala Phe Gly Leu Ser Phe Tyr Ile Leu Leu Asn Leu Gln Asp
                885                 890                 895

Pro Phe Ser Ser Pro Leu Leu Ser Ile Ile Gln Thr Phe Ser Met Met
            900                 905                 910

Leu Gly Asp Ile Asn Tyr Arg Glu Ser Phe Leu Glu Pro Tyr Leu Arg
        915                 920                 925

Asn Glu Leu Ala His Pro Val Leu Ser Phe Ala Gln Leu Val Ser Phe
    930                 935                 940

Thr Ile Phe Val Pro Ile Val Leu Met Asn Leu Leu Ile Gly Leu Ala
945                 950                 955                 960

Val Gly Asp Ile Ala Glu Val Gln Lys His Ala Ser Leu Lys Arg Ile
                965                 970                 975

Ala Met Gln Val Glu Leu His Thr Ser Leu Glu Lys Lys Leu Pro Leu
            980                 985                 990

Trp Phe Leu Arg Lys Val Asp Gln  Lys Ser Thr Ile Val  Tyr Pro Asn
        995                 1000                 1005

Lys Pro  Arg Ser Gly Gly Met  Leu Phe His Ile Phe  Cys Phe Leu
    1010                 1015                 1020

Phe Cys  Thr Gly Glu Ile Arg  Gln Glu Ile Pro Asn  Ala Asp Lys
    1025                 1030                 1035

Ser Leu  Glu Met Glu Ile Leu  Lys Gln Lys Tyr Arg  Leu Lys Asp
    1040                 1045                 1050
```

```
Leu Thr  Phe Leu Leu Glu Lys  Gln His Glu Leu Ile  Lys Leu Ile
    1055                1060                1065

Ile Gln  Lys Met Glu Ile Ile  Ser Glu Thr Glu Asp  Asp Asp Ser
    1070                1075                1080

His Cys  Ser Phe Gln Asp Arg  Phe Lys Lys Glu Gln  Met Glu Gln
    1085                1090                1095

Arg Asn  Ser Arg Trp Asn Thr  Val Leu Arg Ala Val  Lys Ala Lys
    1100                1105                1110

Thr His  His Leu Glu Pro
    1115


<210>  3
<211>  18
<212>  DNA
<213>  Artificial Sequence
<220>
<223>  human TRPA1 primer-F

<400>  3
atgaagtgca gcctgagg                                                 18


<210>  4
<211>  18
<212>  DNA
<213>  Artificial Sequence
<220>
<223>  human TRPA1 primer-R

<400>  4
ctaaggctca agatggtg                                                 18
```